(19) Europäisches Patentamt
European Patent Office
Office européen des brevets

(11) **EP 2 020 910 B1**

(12) **EUROPEAN PATENT SPECIFICATION**

(45) Date of publication and mention
of the grant of the patent:
**10.07.2019 Bulletin 2019/28**

(21) Application number: **07795266.1**

(22) Date of filing: **21.05.2007**

(51) Int Cl.:
*A61B 5/00* (2006.01)   *A61B 1/31* (2006.01)
*A61B 1/07* (2006.01)

(86) International application number:
**PCT/US2007/012359**

(87) International publication number:
**WO 2007/136880 (29.11.2007 Gazette 2007/48)**

(54) **APPARATUS FOR RECOGNIZING ABNORMAL TISSUE USING THE DETECTION OF EARLY INCREASE IN MICROVASCULAR BLOOD CONTENT**

VORRICHTUNG ZUR ERKENNUNG EINES ABNORMALEN GEWEBES MITHILFE DER ERKENNUNG EINER FRÜHEN ERHÖHUNG DES MIKROVASKULÄREN BLUTINHALTS

DISPOSITIF DE RECONNAISSANCE DE TISSU ANORMAL PAR DÉTECTION D'AUGMENTATION PRÉCOCE DANS L'IRRIGATION SANGUINE MICROVASCULAIRE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **19.05.2006 US 801954 P**
**19.05.2006 US 801947 P**
**27.11.2006 US 604659**
**27.11.2006 US 604653**

(43) Date of publication of application:
**11.02.2009 Bulletin 2009/07**

(73) Proprietors:
• **Northshore University Health System**
**Evanston, IL 60201 (US)**
• **Northwestern University**
**Evanston, IL 60208 (US)**

(72) Inventors:
• **BACKMAN, Vadim**
**Chicago, IL 60657 (US)**
• **KIM, Young**
**Skokie, IL 60077 (US)**
• **ROY, Hemant**
**Highland Park, IL 60035 (US)**
• **TURZHITSKY, Vladimir**
**Evanston, Illinois 60201 (US)**
• **LIU, Yang**
**Evanston, IL 60201 (US)**
• **SIEGEL, Michael**
**Seattle, Washington 98105 (US)**
• **WALI, Ramesh**
**Darrien, Illinois 60561 (US)**

(74) Representative: **Beck Greener LLP**
**Fulwood House**
**12 Fulwood Place**
**London WC1V 6HR (GB)**

(56) References cited:
US-A- 5 799 656       US-A1- 2002 135 752
US-A1- 2003 036 751   US-A1- 2003 191 368
US-A1- 2003 236 458   US-A1- 2004 147 843
US-A1- 2004 236 186   US-A1- 2004 242 976
US-A1- 2004 249 274   US-A1- 2006 155 178
US-B1- 6 485 413       US-B1- 6 556 853
US-B1- 6 922 583       US-B2- 6 912 412

**Description**

**FIELD OF THE INVENTION**

The present invention relates generally to light scattering and absorption, and in particular to apparatuses for recognizing possibly abnormal living tissue using a detected early increase in microvascular blood content and corresponding application including in vivo tumor imaging, screening, detecting and treatment, and, in particular, "Early Increase in microvascular Blood Supply" (EIBS) that exists in tissues that are close to, but are not themselves, the lesion or tumor and in tissues that precede the development of such lesions or tumors.

**BACKGROUND OF THE INVENTION**

There are various techniques known for determining abnormality in tissues. Of these techniques, those that are most relevant to the present invention are techniques in which there is detected an increase in blood within tissue that is abnormal. While such techniques have advantages in and of themselves as compared to other methods, they require testing of the abnormal tissue itself which may be difficult to detect. Further, such methods are usable only after the abnormality is sufficiently large, such as a cancerous tissue.

Accordingly, the present invention provides a variety of advantageous optical techniques for assisting in the detection of abnormal tissue, particularly using optical measurements, early in the development of the abnormal tissues themselves.

**SUMMARY OF THE INVENTION**

The invention and preferred embodiments are defined by the appended claims. Further embodiments disclosed herein are for exemplary purpose only. The present disclosure, in one aspect, relates to methods and probe apparatuses and component combinations thereof that are used for examining a target for abnormal tissue, tumors or lesions using what is referred to as "Early Increase in microvascular Blood Supply" (EIBS) that exists in tissues that are close to, but are not themselves, the abnormal tissue and in tissues that precede the development of such lesions or tumors. While the abnormal tissue can be a lesion or tumor, the abnormal tissue can also be tissue that precedes formation of a lesion or tumor, such as a precancerous adenoma, aberrant crypt foci, tissues that precede the development of dysplastic lesions that themselves do not yet exhibit dysplastic phenotype, and tissues in the vicinity of these lesions or pre-dysplastic tissues.

A particular application described herein is for detection of such lesions in colonic mucosa in early colorectal cancer, but other applications are described as well.

In one aspect, the present disclosure describes a method of providing an indication that living tissue within an organ of a body may be abnormal that includes identifying tissue of the organ that contains microvasulature therein, wherein the tissue does not contain the living tissue that may be abnormal and determining from the blood content within the microvasculature whether an early increase in microvascular blood content or supply exists in the tissue to indicate whether the living tissue or nearby tissue may be abnormal.

In another aspect, the present disclosure provides a method of providing an indication that living tissue within an organ of a body may be abnormal comprising the steps of:

Positioning an illumination probe which illuminates the tissue with broadbandlight wherein the illumination probe is disposed in a location that is at a surface of the organ; illuminating, at the location, microvasculature within a tissue of the organ, which tissue does not contain the living tissue that may be abnormal; detecting interacted, light that results from the step of illuminating the location, wherein the detected interacted light is obtained substantially from light scattered from blood in the microvasculature that is within the tissue of the organ that does not contain the living tissue that may be abnormal; estimating at least one of blood content and blood flow in the microvasculature using the detected light; and obtaining the indication that the living tissue may be abnormal using the at least one of estimated blood content and blood flow.

In a further aspect, the steps of illuminating, detecting, and estimating are repeated for a plurality of different locations along the surface and a directional indication of a location of the abnormal living tissue is provided based upon at least some of the plurality of different locations.

In another aspect, the indication is used to decide when to perform another test to determine whether the living tissue within the organ may be abnormal.

In another embodiment there is disclosed an apparatus that emits broadband light obtained from a light source onto microvasculature of tissue disposed within a human body and receives interacted light that is obtained from interaction of the broadband light with the microvasculature for transmission to a receiver. Different further embodiments include combinations of optical fibers, polarizers and lenses that assist in the selection of a predetermined depth profile of interacted light.

In another embodiment, a kit apparatus is described that has various probe tips and/or light transmission elements, such that each different combination preferably provides for a different predetermined depth profile of interacted light.

In a further embodiment, a method of making a spectral data probe for a depth range detection selectivity for detection of blood within microvasculature of tissue is described.

## BRIEF DESCRIPTION OF THE DRAWINGS

These and other aspects and features of the present disclosure will become apparent to those of ordinary skill in the art upon review of the following description of specific embodiments in conjunction with the accompanying figures, wherein:

Fig. 1 illustrates organization of blood supply in colonic mucosa and submucosa.
Fig. 2 shows schematically according to one embodiment a fiber-optic polarization-gated probe: (a) side view and (b) distal (i.e., close to tissue surface) tip.
Fig. 3 shows according to one embodiment photographically a polarization-gated probe in an accessory channel of an endoscope.
Fig. 4 shows according to one embodiment accuracy of optical measurement of Hb content in (a) superficial tissue (obtained from $\Delta I(k)$ using Eq.1) and in (b-d) subsuperficial tissue obtained using Equation (1) (panel (a)) and alternate methods: absorption band area (panel (b)) and absorption band intensity (panel (c)). Is' stands for transport mean free path length. The dashed line shows what the data would look like if the accuracy of measurements were

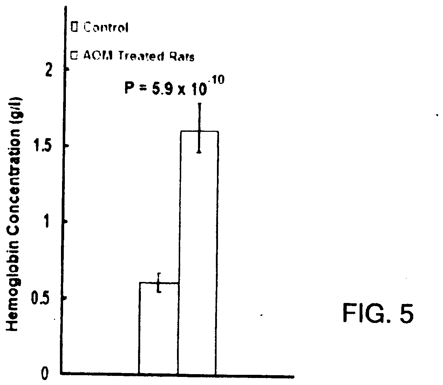

Fig. 5 shows alterations of blood supply in early experimental carcinogenesis observed using polarization-gated signal according to one embodiment of the present disclosure. The data shows EIBS in histologically normal mucosa (i.e., superficial tissue compartment) of AOM-treated rats two weeks after initiation of carcinogenesis by means of AOM injection. This early time point precedes the development of adenomas, aberrant crypt foci and any other currently known markers of colon carcinogenesis. EIBS was observed only in the distal colon of AOM-treated rats and no blood content increase was found in the proximal colon, consistent with the fact that precancerous and cancerous lesions develop primarily in the distal colon in this model.

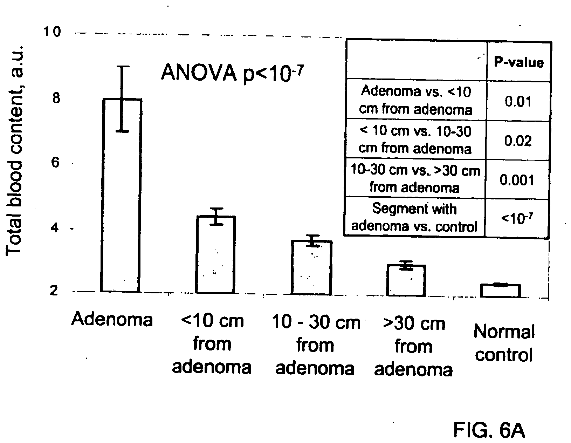

Figs. 6(a)-6(d) show schematically according to one embodiment of the present disclosure the observation of EIBS in our *in vivo* studies (n=196 patients). The x-axis shows a location of EIBS reading in relation to the location of an adenoma. Normal control values were taken from patients with negative colonoscopy from the same colonic segments where adenomas were found in patients with positive colonoscopy. (a) EIBS from total blood content; (b) EIBS in superficial tissue (e.g. mucosa) extends >30 cm, i.e. EIBS can be observed in colonic segments other than the one where an adenoma is located; (c) EIBS in subsuperficial tissue (e.g. mucosa and superficial mucosa) extends <30 cm from the location of an adenoma; (d) Benign, hyperplastic polyps do not lead to EIBS outside extend of a polyp.
Fig. 7 shows the data confirming the phenomenon of EIBS in AOM-treated rats by Western blot. Although Western blot clearly shows EIBS 8 weeks after initiation of carcinogenesis (i.e., aberrant crypt foci (ACF), pre-adenoma stage of colon carcinogenesis), the sensitivity and accuracy of Western blot was not sufficient to measure EIBS in pre-ACF and stage (two weeks after initiation of carcinogenesis). For comparison, the disclosed optics approach has sensitivity sufficient enough to detect EIBS at this earliest time point.
Fig. 8 shows according to one embodiment a methodology of EIBS-assisted colonoscopy.
Fig. 9 illustrates a probe kit
Figs 10(a)-(j) illustrate various configurations of the probe.
Fig. 11 illustrates an overall system.
Figs. 12(a)-(b) illustrate various embodiments of an indicator device.

## DETAILED DESCRIPTION OF THE PREFERRED EMBODIMENTS

The present disclosure is more particularly described in the following examples that are intended as illustrative only since numerous modifications and variations therein will be apparent to those skilled in the art. Various embodiments of the disclosure are now described in detail. Referring to the drawings, like numbers indicate like components throughout the views, As used in the description herein and throughout the claims that follow, the meaning of "a", "an", and "the" includes plural reference unless the context clearly dictates otherwise Also, as used in the description herein and throughout the claims that follow, the meaning of "in" includes "in" and "on" unless the context clearly dictates otherwise.

Moreover, titles or subtitles may be used in the specification for the convenience of a reader, which shall have no influence on the scope of the present invention. Additionally, some terms used in this specification are more specifically defined below.

The terms used in this specification generally have their ordinary meanings in the art, within the context of the invention, and in the specific context where each term is used. Certain terms that are used to describe the invention are discussed below, or elsewhere in the specification, to provide additional guidance to the practitioner regarding the description of the invention. For convenience, certain terms may be highlighted, for example using italics and/or quotation marks. The use of highlighting has no influence on the scope and meaning of a term; the scope and meaning of a term is the same, in the same context, whether or not it is highlighted. It will be appreciated that same thing can be said in more than one way. Consequently, alternative language and synonyms may be used for any one or more of the terms discussed herein, not is any special significance to be placed upon whether or not a term is elaborated or discussed herein. Synonyms for certain terms are provided. A recital of one or more synonyms does not exclude the use of other synonyms. The use of examples anywhere in this specification including examples of any terms discussed herein is illustrative only, and in no way limits the scope and meaning of the invention or of any exemplified term. Likewise, the invention is not limited to various embodiments given in this specification.

Unless otherwise defined, all technical and scientific terms used herein have the same meaning as commonly understood by one of ordinary skill in the art to which this invention pertains. In the case of conflict, the present document, including definitions will control.

As used herein, "around", "about" or "approximately" shall generally mean within 20 percent, preferably within 10 percent, and more preferably within 5 percent of a given value or range. Numerical quantities given herein are approximate, meaning that the term around", "about" or "approximately" can be inferred if not expressly stated.

The present disclosure, in one aspect, relates to a method for examining a target for tumors or lesions using what is referred to as "Early Increase in microvascular Blood Supply" (EIBS) that exists in tissues that are close to, but are not themselves, the lesion or tumor. While the abnormal tissue can be a lesion or tumor, the abnormal tissue can also be tissue that precedes formation of a lesion or tumor, such as a precancerous adenoma, aberrant crypt foci, tissues that precede the development of dysplastic lesions that themselves do not yet exhibit dysplastic phenotype, and tissues in the vicinity of these lesions or pre-dysplastic tissues.

A particular application described herein is for detection of such lesions in colonic mucosa in early colorectal cancer ("CRC"), but other applications are described as well.

The target is a sample related to a living subject such as a human being or animal. The sample may be a part of the living subject such that the sample is a biological sample, wherein the biological sample may have tissue developing a cancerous disease.

The neoplastic disease is a process that leads to a tumor or lesion, wherein the tumor or lesion is an abnormal living tissue (either premalignant or cancerous), such as pancreatic cancer, a colon cancer, an adenomatous polyp of the colon, a liver cancer, a lung cancer, a breast cancer, or other cancers.

The measuring step is preferably performed in vivo, though it can be performed ex vivo as well. The measuring step may further comprise the step of acquiring an image of the target. The image, obtained at the time of detection, can be used to later analyze the extent of the tumor, as well as its location. Measuring of blood content using interacted light, which can include scattering as well as other optical methods, can include insertion of a probe for in-vivo usages in which blood content and/or flow is measured in tissue of a solid organ. Also, the present invention can be used to insert a probe into a body cavity, such as for measurements of tissues that are in the GI tract, respiratory track, or the like.

In one embodiment, the method comprises projecting a beam of light, such as broadband light, to a target that has tissues with blood circulation therein. As used herein, broadband light is light having at least two different wavelengths. At least one spectrum of light scattered from the target is then measured, and blood supply information related to the target is obtained from the measured at least one spectrum. The obtained blood supply information comprises data related to at least one of blood content, blood oxygenation, blood flow and blood volume.

The method according to an embodiment may include obtaining a first set of the blood supply information from a first location of the target and then obtaining a second set of the blood supply information from a second location of the target. The first set of the blood supply information at a first location of the target and the second set of the blood supply information at a second location of the target can then be compared to determine the status of the target. One can compare the data to indicate whether the tumor or lesion exists at all by comparison to previously established microvascular blood content values from patients who harbor neoplasia and from those who are neoplasia free. The data can also indicate whether the tumor or lesion is closer the first or second location by comparison of the blood content values from the first and second locations. Rather than measuring blood content in a given tissue site, at least two but preferably more tissue sites may be assessed located within a given area of tissue and the statistical properties of blood content or blood flow distribution can be determined for this area to determine the status of the target. For example, the maximal blood content within an area can be used to determine the status of the target. Other statistical measures include mean, average, median, standard deviation, maximal value, and minimal value.

Rather than having different locations, the same location can be compared at different times, days, months or years apart, to determine the status of the target, and in particular whether the tumor or lesion has developed or if it previously existed whether it has gotten larger.

The present disclosure, in another aspect, relates to an apparatus for examining a target. In one embodiment, the apparatus comprises a light source such as, for example, a light emitting device configured and positioned to project a beam of light to a target; and means for measuring at least one spectrum of light scattered from the target; and means for obtaining blood supply information related to the target from the measured at least one spectrum.

The apparatus may further comprise a detector that obtains a first set of the blood supply information at a first location of the target. The same detector can be used to obtain a second set of the blood supply information at a second location of the target. An algorithm, which is executed by a controller or computer, analyzes that data is used to determine the status of the target, typically by comparing the first set of the blood supply information at a first location of the target and the second set of the blood supply information at a second location, although comparisons against reference blood supply information (that does or does not suggest the presence of a tumor or lesion) can be used as well. This same apparatus can be used to implement the method mentioned above where the same location is sampled at different points in time.

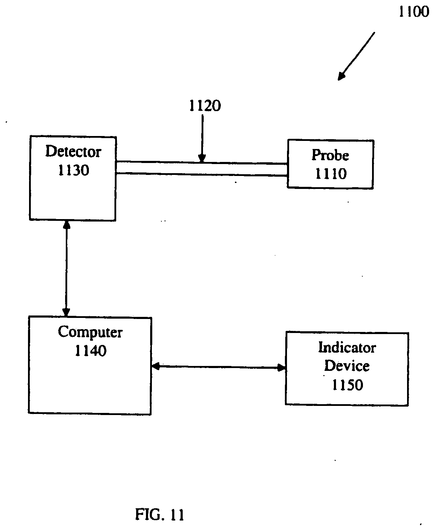

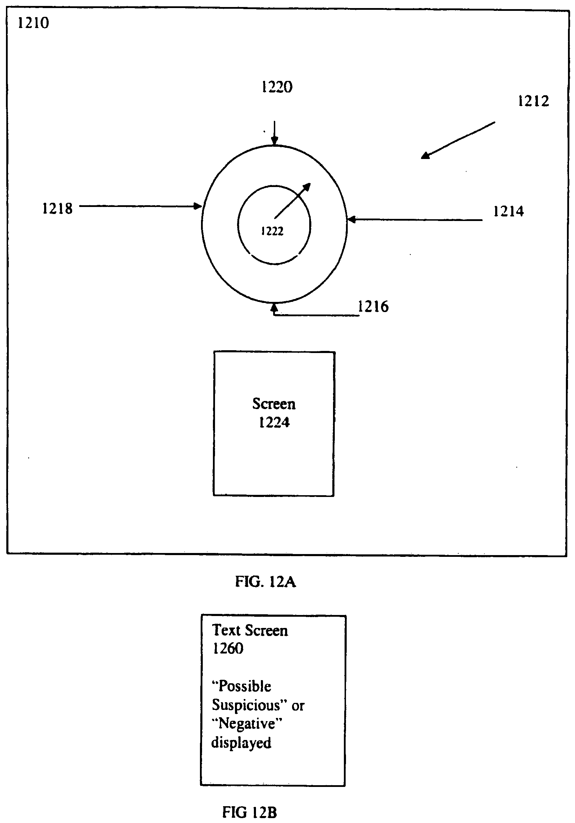

The overall system 1100, as shown described herein and also shown in Fig. 11, includes the probe 1110 (or probe combined with other diagnostic or surgical instruments as described herein), the fiber-optic cable system 1120, the detector 1130, and a computer 1140 (or other type of controller) that is used to analyze the detected data, as described elsewhere herein, and may further include an indicator or alert device 1150 such as, for example, a display device 1210 as shown in Fig. 12(a) for visually indicating the actual or relative measurements of a blood supply characteristic of at least one of blood content and blood flow. The display device 1210 shown in Fig. 12(a) includes an indicator 1212 with an arrow 1222 that can point to positions 1214 right, 1216 down, 1218 left and 1220 up, and which can also point to positions between these, to indicate the direction from the current position that a probe should be moved to assist in determining the present of abnormal tissue, and/or a display screen 1224 that can provide various blood content information. In this embodiment, the indicator 1212 will typically be useful after at least two readings occur in different locations of the tissue being tested. It is apparent that the indicator 1212 can be made part of a screen 1224 as well.

Suitable display screens, which can also include the indicator, may also include electronic monitors such as liquid crystal displays (LCD), cathode ray tube (CRT) displays, plasma displays, panels containing light emitting diodes (LEDs) or other technologies for alerting a user. In one embodiment, the display screen 1222 preferably uses an on-screen menu that a user is able to control in order to label the recorded data based on a colonic segment from which the data are being recorded and also record colonoscopy finding by clicking on an on-screen button. The spectral data is analyzed, as discussed herein, substantially immediately after a signal is being acquired. (The signal acquisition time is - 50 milliseconds.) As such, the algorithm executed by the computer in software uses the spectral data acquired by the probe to compute blood content values in "superficial" and "subsuperficial" tissue as described herein. The EIBS parameters are used by the algorithm to determine if blood content is increased above the normal range. The on-screen menu also preferably enables an operator to enter before or during the procedure demographic, personal and family history characteristics including patient's age, gender, body mass index, smoking history (yes, no, quit), history of alcohol, history of prior colonoscopy findings (adenoma/adenocarcinoma/no neoplasia on previous colonoscopy, and the use of anti-inflammatory medications. These characteristics can be used by the algorithm to include into the diagnosis. After a measurement, the on-screen display provide an "indicator of diagnosis". In principle, an indicator of diagnosis can be one of a number types including a text message, a color sign, a sound, etc.

In a specific embodiment as shown in Fig. 12(b), the indicator device uses a screen 1260 that displays one of the following text messages: "positive, suspicious region" if the diagnostic algorithm indicates that an adenoma is likely to be located in the vicinity of the tissue sites assessed for EIBS, or message "negative", if this segment of the colon is most likely adenoma-free.

Thus, the display device may further indicate by graphs, symbols, color, alphanumerics, light and similar techniques and combinations thereof the presence of increased blood supply and/or a comparison of consecutive or non-consecutive measurements of a characteristic of increased blood supply. It should be readily understood that indicator devices according to the present invention include for example, sound emitting devices, vibration generating devices for vibrating instruments to alert the user or any other hardware or software technique for alerting the user regarding an actual or relative measurements of a blood supply characteristic of at least one of blood content and blood flow, suggested direction for movement of the probe and/or likely proximity of abnormal tissue.

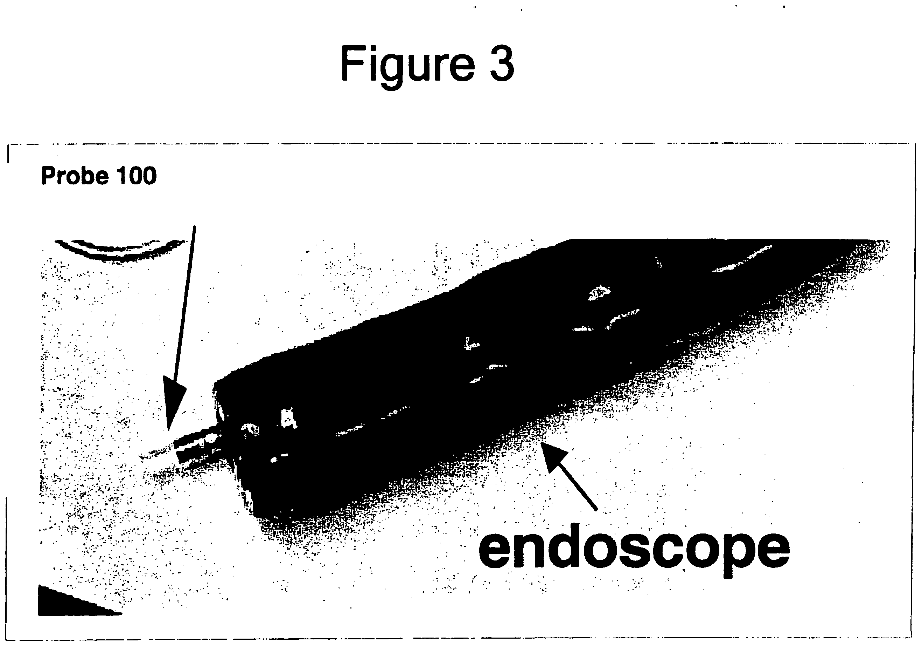

The Fig. 3 embodiment illustrates the probe in use with an endocscope. This is exemplary and the probe can be inserted into or contained or moveable within other surgical or diagnostic instruments, such as a sigmoidoscope for a sigmoidoscopy where the tissue is a colon and a larger portion of a colon is analyzed than would be possible using solely the sigmoidoscope. In this use, the larger portion of the colon that can be analyzed is at least another 10-30 cm of the colon. A rectoscope can also be used, which also allows 10-30 cm of the colon to be analyzed, and removes the need for a sigmoidoscopy.

For many of the procedures herein, when moving the probe, different locations are separated by a distance of greater than 5cm or 10cm.

A superficial and subsuperficial polarization and spectral data analysis algorithm that allows for discrimination between spectral data obtained from the mucosal and the submucosal tissue is described in the following Equation 1. It is noted that this algorithm, because it is based on the quantitative analysis of spectroscopic signals recorded as a result of elastic scattering and absorption of light in tissue without the need for tissue biopsy or any other preparation, allows for almost real-time processing of the polarization gated signals, which makes it very useful for clinical screening applications.

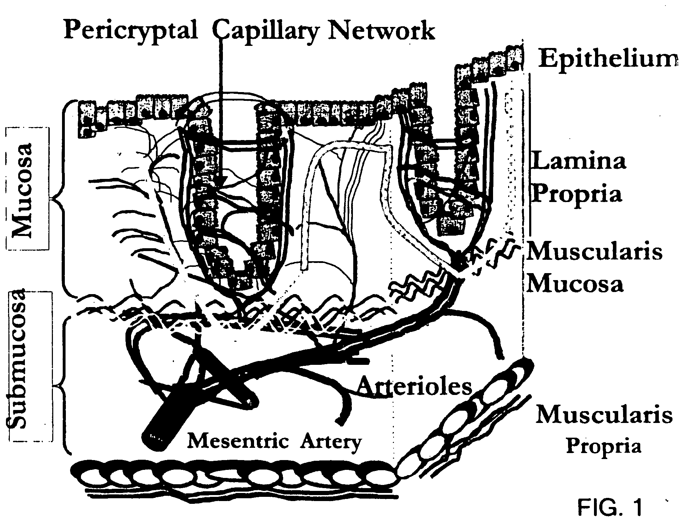

Polarization gating enables assessment of blood content in several tissue compartments at the same time. The two principal tissue compartments that are being analyzed for the blood content are the "superficial" (e.g. mucosal) and "subsuperficial" (e.g. mucosal and submucosal) tissues (Fig. 1).

In order to assess blood content and/or blood flow in superficial tissue, polarization-gated spectrum is preferably used. Blood content in subsuperficial tissue can be measured by using co-polarized spectrum, arbitrarily polarized (also referred to as unpolarized or total) spectrum (which is the sum of co-polarized and cross-polarized signals), or cross-polarized spectrum. These three signals have progressively deeper depths of penetrations. The depth of penetration of these signals can be selected by the instrumentation design in order to selectively probe mucosal and mucosal/submucosal tissue for a given organ and tissue type. The polarization-gated signal $S(\lambda)$ is taken as the difference between copolarized and crosspolarized signals, each normalized by the corresponding copolarized and crosspolarized spectra from a polytetrafluoroethylene reflectance standard (Ocean Optics). Co-polarized, arbitrarily polarized (also known as unpolarized) and cross-polarized signals ($D(\lambda)$) are normalized accordingly. In the teachings that follow, as an example, it is considered that subsuperficial blood content is measured from the cross-polarized signal. It should be understood, however, that similar analysis can be performed based on co-polarized and arbitrarily polarized signals. Thus, in principle, blood content can be measured for four different depths of penetration.

In both cases of superficial and subsuperficial blood content, it is assumed that the variability in path length due to differences in optical properties within the sample is small. While it is known that Beer's law cannot be directly applied to the analysis of scattered light because of unknown attenuation due to scattering, Beer's law served as the starting point for the analysis, as attenuation due to absorption has an inverse exponential relationship with absorber concentration. This assumption can be expressed as follows:

$$S(\lambda) = S_{scattering}(\lambda) \exp[-L_S (\alpha_{HbO2} A^{HbO2}(\lambda) + \alpha_{Hb} A^{Hb}(\lambda))] \qquad (1)$$

$$D(\lambda) = D_{scattering}(\lambda) \exp[-L_D (\beta_{HbO2} A^{HbO2}(\lambda) + \beta_{Hb} A^{Hb}(\lambda))],$$

where $S_{scattering}(\lambda)$ and $D_{scattering}(\lambda)$ represent light scattering signals from the superficial and subsuperficial layers of a sample, respectively, if it were devoid of absorbers. $A(\lambda)$ represents the absorption spectrum of all of the absorbers present (oxygenated and deoxygenated hemoglobin), coefficients $L_S$ and $L_D$ represent the path lengths for polarization-gated and cross-polarized signals, and coefficients $\alpha$ and $\beta$ represent the absorbers' concentrations for superficial and subsuperficial tissue depths. To account for different contributions of oxygenated and deoxygenated hemoglobin, two coefficients are used: $\alpha_{HbO2}$ and $\alpha_{Hb}$ in case of superficial blood content and $\beta_{HbO2}$ and $\beta_{Hb}$ in case of subsuperficial blood content. Similar analysis can be performed for co-polarized and arbitrarily polarized (i.e., total, unpolarized) spectra.

Spectra for deoxygenated and oxygenated blood content can be measured in a hemoglobin solution in water. The solution is placed in a glass-bottomed culture slide directly on top of a reflectance standard and measured to obtain a spectrum $A^{HbO2}(\lambda)$. It is then deoxygenated by adding sodium dithionite to measure the $A^{Hb}(\lambda)$. $L_S$ and $L_D$ are determined in the process of initial instrument calibration in tissue models. The remaining unknowns for the analysis are $S_{scattering}(\lambda)$ and $D_{scattering}(\lambda)$. To fill this gap it is assumed that expected $S_{scattering}(\lambda)$ and $D_{scattering}(\lambda)$ should have a smooth decreasing spectral line shape between $\lambda = 480$ and $680$ nm, thereby lacking spectral features of hemoglobin absorption, which include absorption bands at 542 and 576 nm in the case of oxygenated blood and 555 nm in the case of deoxygenated blood. In particular, second-order decreasing polynomial or inverse power-law spectral line shapes can be used as target line shapes with essentially the same results. Over this narrow spectral range, this assumption is reasonable. Thus the superficial and subsuperficial polarization and spectral data analysis algorithm tests values of $\alpha_{HbO2}$ and $\alpha_{Hb}$ for superficial and $\beta_{HbO2}$, and $\beta_{Hb}$ for subsuperficial tissue over the range of interest and finds those that provide the best agreement between the resulting $S_{scattering}(\lambda)$ and $D_{scattering}(\lambda)$ and a target line shape in the least-squares sense. This process can be continued iteratively.

Once coefficients $\alpha_{HbO2}$ and $\alpha_{Hb}$ are found, a number of other related metrics characterizing blood content can be found including

$$\text{total blood content} = \alpha_{HbO2} + \alpha_{Hb} \text{ and} \qquad (2)$$

$$\text{oxygen saturation} = \alpha_{HbO2}/(\alpha_{HbO2} + \alpha_{Hb}).$$

The validity of all equations was verified using tissue phantom experiments.

In one embodiment, at least one spectrum of light scattered from the target is measured by a fiber optic probe, wherein the fiber optic probe comprises a polarization-gated fiber optic probe configured to detect the blood supply information. The light source comprises an incoherent light source (such as a xenon lamp).

In one embodiment, the fiber optic probe includes a proximal end portion, an opposite, distal portion, and a body portion with a longitudinal axis defined between the proximal end portion and the distal portion. The body portion is formed with a cavity along the longitudinal axis. At least one first type of fiber is used for delivering a beam of energy to a target, wherein the at least one first type fiber is at least partially positioned within the cavity of the body portion. An optical element is positioned at the proximal end portion and configured to focus the beam of energy to the target. At least one second type fiber is used for collecting scattered energy from the target, wherein the at least one second type fiber is at least partially positioned within the cavity of the body portion.

The fiber optic probe may further comprise at least one linear polarizer optically coupled to the at least one first type fiber and the at least one second type fiber and positioned proximate to the proximal end portion, and wherein the optical element is positioned at the proximal end portion and configured to focus the scattered energy from the target to the at least one linear polarizes for the at least one second type fiber to collect.

The optical element comprises at least one of a ball lens, a graded refractive index lens, an aspheric lens, cylindrical lens, convex-convex lens, and plano-convex lens, although preferably just a single lens is used. Lenses or any combinations of them other than these above-mentioned lenses can also be used. It is further noted that different lenses can be used to assist in discriminating measurements and to achieve different tissue penetration depths. Thus, for example, to achieve the shortest penetration depth, a lens can be positioned at the focal distance from the end of the light-collecting fibers with the fibers positioned symmetrically around the axis of the lens. This configuration further increases the intensity of collected light, particularly when a probe is at a distance form tissue, and provides improved stability of the signals collected by the probe in terms of different distances from tissue (if a probe is not in contact with tissue) and pressures exerted by the probe onto tissue (if a probe is in contact with tissue). Shorter penetration depth can also be achieved by using a lens with a shorter focal distance, smaller numerical aperture of the illumination and/or collection fibers, and larger distance between illumination and collection fiber. In principle, penetration depths from a few tens of microns to a few millimeters can be achieved by choosing a proper combination of these probe characteristics.

The at least one first type fiber comprises an illumination fiber, wherein the illumination fiber is optically coupled to the light source.

The at least one second type fiber can also be formed with one or more collection fibers, wherein the one or more collection fibers are optically coupled to an imaging spectrograph and a CCD at the distal end portion, which imaging spectrograph is used to obtain an image of the target. The body portion comprises a tubing.

The following further details of the preferred embodiments, will further describe the invention. As will become apparent, a substantial part of the following disclosure relates to the EIBS phenomenon as applied to determination of the presence of colonic neoplasia (adenomatous polyp or carcinoma) through analysis of colonic tissue remote to the lesion and as a guide to the determination of the location of a tumor or lesion with the colon. This disclosure is also applicable to detection of tumors or lesions within other organs, and to the extent variations exist with respect to such detection in different organs, such is noted.

Although it has been well established that blood supply to tumor tissue is increased, very little attention has been given to alterations in blood supply at the pre-neoplastic stage and histologically/endoscopically normal appearing mucosa outside the extend of a neoplastic or pre-neoplastic lesion, largely due to the methodological difficulties in reliably quantitating microvascular blood supply. EIBS is most pronounced in the very superficial mucosa (peri-cryptal capillary plexus). This makes up a very small amount of total colonic microcirculation. The reason polarization-gated optical spectroscopy can detect this is that it can specifically and accurately analyze this plexus.

It is further noted that the present disclosure can distinguish between benign and malignant tumors, such that when a tumor is seen, looking at the surrounding normal mucosa can assist in distinguishing a hyperplastic (benign) from an adenomatous (premalignant) tumor. Abnormal blood supply in the microscopically normal mucosa adjacent to the lesion-- EIBS -- will be seen in adenomatous but not hyperplastic polyps. Furthermore, previous angiogenesis studies focused on blood supply increase to a neoplastic lesion itself. EIBS manifests itself as an increase in blood supply in the microcirculation (primarily mucosa) supplying blood to epithelium. EIBS occurs very early during the process of colon carcinogenesis. Our data in animal models of colon carcinogenesis showed that EIBS starts at earlier than development of adenomas and aberrant crypt foci (i.e. the earliest marker of carcinogenesis) and precedes the development of currently known molecular markers of colonic neoplasia. Furthermore, EIBS can be detected outside a neoplastic lesion. This allows for detecting a lesion outside its physical extent, as discussed by the examples and further disclosure provided below.

Examples

Without intent to limit the scope of the invention, exemplary instruments, apparatus, methods and their related results according to the embodiments of the present invention are given below. Note that titles or subtitles may be used in the examples for convenience of a reader, which in no way should limit the scope of the invention. Moreover, certain theories are proposed and disclosed herein; however, in no way they, whether they are right or wrong, should limit the scope of the invention so long as the invention is practiced according to the invention without regard for any particular theory or scheme of action.

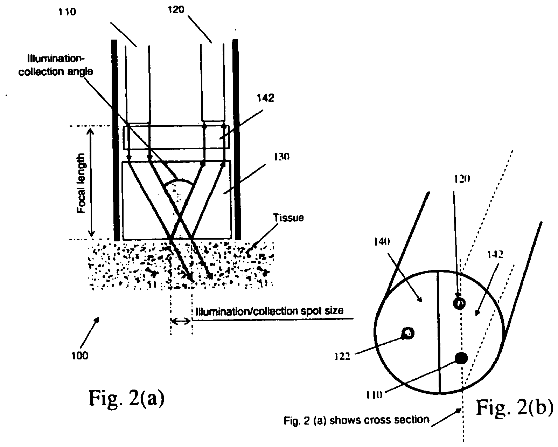

*Polarization Gated Fiber-Optic Probe to Detect EIBS: Polarization Gated Fiber-Optic Probe to Detect EIBS:* In one aspect, a fiber-optic probe has been developed to accurately detect blood supply in tissue mucosa. Figures 2A and 2B illustrate the design of the probe and Fig. 3 shows a photograph of the probe protruding from an accessory channel of a colonoscope. The probe 100 has one or more 100 $\mu$m-diameter fibers, one delivery fiber 110 used for delivery of linearly polarized light from a Xe-lamp (not shown) onto the tissue surface and the other two fibers 120 and 122 for collecting scattered light from the tissue. A positive lens 130 was positioned at the focal distance from the fiber tips. Several lens types were also tested, including ball, graded refractive index (GRIN), and aspherical lenses. All of the different types of lenses could be used and these provide different performance of the probe in terms of the depth of penetration. In the configuration where the lens 130 was positioned at the focal distance from the fiber tips, it focused light backscattered from a sample onto different fibers 120 and 122, depending on the angle of backscattering. It also ensured that all collection fibers receive scattered light from the same tissue site, which coincides with the illumination spot. The lens 130 does not have to be positioned at the focal distance from the fibers 110, 120, 122, but this configuration provides better performance in terms of 1) shorter penetration depth, in particularly for the polarization gated signal, 2) increases signal level and, thus, time required to collect the signal with sufficient signal-to-noise ratio, 3) prevents collection of specular reflection from probe and tissue surfaces, and 4) improves stability of the measurements in terms of probe displacement from tissue surface in non-contact geometry or the pressure exerted by the probe onto a sample. In the proximal end of the probe 100, the collection fibers 120, 122 are coupled to an imaging spectrograph and a CCD. Two thin film polarizers 140, 142 were mounted on the proximal tip of the probe to polarize the incident light and enable collection of both polarization components (i.e. parallel I∥ and I⊥ perpendicular to the incident polarization) of the backscattered light to allow for polarization gating. All components of the probe 100 were made from FDA approved materials.

A lens at the probe tip allows selecting a desired penetration depth. For example, to achieve a shorter penetration depth, a lens can be positioned at the focal distance from the end of the fibers with the fibers positioned symmetrically around the axis of the lens. Furthermore, one can use a lens with a shorter focal distance, smaller numerical aperture of the illumination and/or collection fibers, and a larger distance between the illumination and collection fiber. For example, probes were fabricated with a GRIN lens with the penetration depth in colon tissue for polarization-gated signal ~85 microns (~ 1.7 mean free path lengths) and that for cross-polarized light ~260 microns. A ball lens probe with penetration depths ~23 and 275 microns was also developed. As such, it is apparent that penetration depths from a few tens of microns to a few millimeters can be achieved by choosing a proper combination of these probe characteristics.

*Polarization Gating:* Polarization gating has been previously used to selectively record short-traveling photons as well as to increase contrast for photons emerging from deeper tissue. As has been shown by our group, the differential polarization signal $\Delta I(\lambda)$ = I∥ $(\lambda)$ - I⊥ $(\lambda)$ is primarily contributed by scatterers located close to the tissue surface and, therefore, particularly sensitive to the properties of the superficial tissues, e. g. epithelial. Our experiments showed that the contribution to the differential polarization signal from deeper tissue structures decreases exponentially with "optical distance" to the structure and, hence, with depth ($\tau$=L/ls with $L$ "physical" depth and ls photon mean free path length in tissue). Because optical density of epithelium is much smaller than that of underlying connective tissue, in the colon, differential polarization signals are primarily collected from the epithelium plus up to ~50 $\mu$m of underlying connective tissue. This near-surface portion of subepithelial stoma contains a network of capillaries supplying oxygen to the epithelium. Co-polarized signal I∥, arbitrarily polarized signal I∥ + I⊥ and cross-polarized signal I⊥ contain information about progressively deeper tissue, up to several millimeters below the surface for certain probe configurations.

*Measurement of Superficial Blood Content* The blood content in the capillaries immediately below epithelium can be quantitatively estimated from the spectral analysis of $\Delta I(A)$. We developed several methods of spectral data analysis. The following example [Eq. 3] discusses an earlier version of the method that provides for analysis of superficial blood content. A more recent version [Eqs. 1 & 2] that provides improved accuracy of blood content estimation in both the superficial and subsuperficial mucosa is discussed above.

With respect to this earlier version, it operates based on a determination of blood content values that include effects of both scattering and absorption by red blood cells in the microvasculature and model the presence of absorption bands in the spectra due to both light absorption and scattering by blood.

We obtained the scattering images of rats' red blood cells (RBCs). Although Hb primarily absorbs visible light, it is not sufficient to measure only the absorption spectra of Hb molecules. RBCs, which are filled with Hb, are large scatterers approximately 7-8 microns in diameter. Therefore, the contribution from the RBCs couples both absorption and scattering. Our data demonstrate that differential polarization signal measured from a tissue $\Delta I(\lambda)$ can be written as

$$\Delta I(\lambda) = \Delta Is(\lambda) + \alpha \Delta I_{RBC}, \tag{3}$$

where $\Delta Is(\lambda)$ is the signal contributed by epithelial cells and other non-RBC components of the superficial tissue (not a priori known), $\Delta I_{RBC}$ is the signal experimentally measured from isolated ref blood cells (thus, this signal is known), and $\alpha$ is the number density of RBCs per mm$^2$. This early version of the polarization and spectral data analysis algorithm for superficial blood content was used to find the fitting parameter $\alpha$ by minimizing the Hb absorption bands in $\Delta Is(\lambda)$. This early version of the polarization and spectral data analysis algorithm, rather than using an exponential attenuation of light propagating in tissue by the tissue blood content that was used in Eq. 1, relies upon a linear calculation where the contribution from the red blood cells is assumed to be additive to that of tissue scattering; this contribution includes both light absorption and red blood cell scattering.

For *in situ* applications, where hemoglobin is present in both oxygenated ($\Delta I_{RBC}\text{-}_{O2}(\lambda)$), and deoxygenated ($I_{RBC}\text{-}_{O2}(\lambda)$) forms,

$$\Delta I_{RBC}(\chi;\lambda) = \chi \, \Delta I_{RBC}\text{-}_{O2}(\lambda) + (1 - \chi) \, I_{RBC}\text{-}_{O2}(\lambda), \tag{4}$$

with x the oxygen saturation coefficient also determined by means of optimization.

*Measurement of Subsuperficial Blood Content.* We also assessed blood supply in the deeper tissue layers, i.e. mucosa and submucosa, via $I\perp(\lambda)$ (as opposed to $\Delta I$, this signal is primarily contributed not by single but multiple scattering process). For subsuperficial blood content, we developed several methods of spectral data analysis. The following example in Eq. 5 discusses an earlier version of the method. A more recent version that provides improved accuracy of blood content estimation is discussed above in Eqs. 1 & 2.

The changes in the blood supply to mucosa/submucosa is detected, as noted, by means of the analysis of the cross-polarized signal $I\perp(\lambda)$. Briefly, a diffusion approximation model is fit to the data. The model $I_M$ depends on the spectra of the transport scattering $\mu'_s(\lambda)$ and absorption coefficient

$$\mu_\alpha(\lambda) = \chi \, \mu_{\alpha\text{-}02}(\lambda) + (1 - \chi) \, \chi \, \mu_{\alpha\text{-}D02}(\lambda), \tag{5}$$

which is contributed by both oxygenated $\mu_{\alpha\text{-}02}$ and deoxygenated $\mu_{\alpha\text{-}D02}$ Hb species with oxygen saturation $\chi$ found as a fitting parameter $\mu_\alpha$ is proportional to the concentration of the respective form of Hb in tissue. It is conventionally assumed that Hb is the only significant absorber of visible light in the mucosa and $\mu'_s(\lambda)$ should not exhibit Hb absorption bands.

This diffusion approximation-based, early version of the algorithm requires substantial processing time (on the order of several minutes per sample), rather than the nearly real-time results from the algorithm of Eq. 1 discussed above. The reason for such an improvement is because the algorithm of Eq. 1 does not require the use of a diffusion approximation that is computationally intensive.

*Measurement of Oxygen Saturation.* As discussed above, due to distinctly different absorption spectra of oxy- and deoxy-hemoglobin, not only does spectral analysis of polarization gated signals enable measurement of blood content but also blood oxygenation (aka. oxygen saturation, $S_{O2} = \chi$. We validated $S_{O2}$ calculations from spectral data. The accuracy of oxygen saturation measurement was excellent with error <1%.

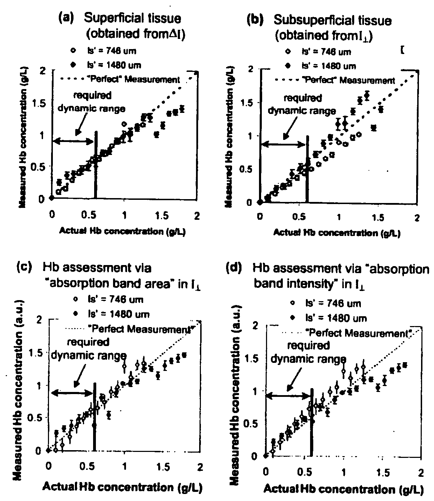

*Accuracy of EIBS Assessment Using Fiber-Optic Probe.* We also validated the ability of the probe to assess hemoglobin concentration in studies with tissue models. The tissue models were fabricated and the analysis of spectral data was performed as discussed above. As shown in Fig. 4, the probe enables accurate assessment of hemoglobin concentration. The standard error of measurements for concentrations <12 g/L for superficial tissue was <0.01 g/L and that for deeper tissue was <0.02 g/L. We point out that according to EIBS data in animals as well as humans, the dynamic range of Hb concentrations was well within this range. Thus, the probe provides sufficiently accurate measurement of blood content in physiologic range with error of measurement sufficient to reliably identify EIBS.

It is also possible to measure blood content based on the analysis of the area of hemoglobin absorption spectral band and/or the maximum of this absorption band. As shown in Figs. 4(c,d), both "absorption band area" and "absorption band intensity" methods enable accurate assessment of hemoglobin concentration with the 10 error of measurements for concentrations <1.2 g/L 0.02 g/L and 0.03 g/L, respectively, and for concentrations from 1.2 to 18 g/L, 0.07 g/L and 0,09 g/L, respectively.

*EIBS Precedes Formation of Known Markers of Colon Carcinogenesis.* EIBS precedes the development of any currently known histologic or molecular markers of colon carcinogenesis.

Specifically, we assessed blood content in the colons of rats treated with a colon specific carcinogen, azoxymethane (AOM). The AOM-treated rat model is one of the most robust and widely used animal models of CRC. As in humans, in this model, neoplasia progresses through a well defined sequence of events with the exception that the time course of carcinogenesis in the AOM-treated rat model is much faster than the one in humans: the earliest detectable marker of carcinogenesis, ACF, develops in 4-12 weeks after AOM injection, adenomas are observed in 20-30 weeks, and carcinomas develop after 40 weeks. No histologic, molecular or genetic markers have been shown to allow earlier diagnosis (<4-12 weeks). As shown in Fig. 5, our data demonstrate that EIBS occurs as early as 2 weeks after AOM-injection (p-value$<10^{-9}$). Importantly, EIBS was detected only in the distal colon and not in the proximal colon (p-value$<10^{-11}$). This mirrors the progression of carcinogenesis in the AOM-treated rat model as AOM induces carcinogenesis primarily in the distal colon with only minimal effect on the proximal colon, as has been validated by numerous studies and our data as well.

*EIBS Is an Accurate Predictor of Colonic Neoplasia: Animal Study.* In order to assess whether EIBS may serve as a clinically useful biomarker, we determined the performance characteristics of EIBS to detect future ACF in AOM-treated rats. It was found that EIBS had excellent ability to distinguish animals at risk for CRC from the negative controls even at the pre-ACE stage of CRC, two weeks after AOM treatment. Indeed, the diagnostic accuracy of EIBS far exceeded conventional markers with high (>90%) sensitivity, specificity, positive and negative predictive values even at the earliest stages of colon carcinogenesis, preceding the development of currently known markers of CRC (Table 1).

**Table 1: EIBS diagnosis of predisposition to CRC in AOM-treated rat model**

| Sensitivity | 94% |
|---|---|
| Specificity | 96% |
| PPV | 97% |
| NPV | 92% |

*EIBS Gradient Localizes Adenomas: In Vivo Clinical Study.* To prove that EIBS and, importantly, EIBS gradient (i.e., progressive increase in blood content towards an adenoma) can be observed in vivo. A pilot investigation was conducted in human subjects undergoing screening colonoscopies (196 patients including 48 with adenomas out of which 43 were diminutive and 5 advanced, 27 subjects with hyperplastic benign polyps, and 121 patients with negative colonoscopies). We used an endoscopically compatible fiber-optic probe discussed in the preceding section. The probe was inserted into the accessory channel of a colonoscope. During colonoscopy, EIBS spectral data were acquired by the probe from the following locations: adenomatous polyp (if present), an endoscopically normal location within 10 cm from the adenoma, from the same colonic segment where the adenoma was located (typically within 30 cm from the adenoma) and the other segments (dubbed "outside" segments).

In patients with negative colonoscopy, measurements were taken at random from each of the three colonic segments (i.e., descending colon including rectum and sigmoid colon, mid-transverse colon, and ascending colon including the cecum). On average, three spectra were obtained from each tissue site and more than 10 different tissue sites were probed for each patient.

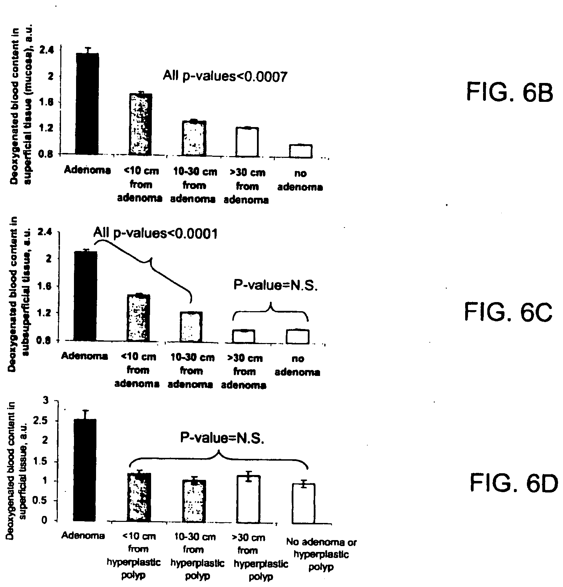

Our data (Fig. 6) demonstrate a marked augmentation of blood content in the uninvolved (endoscopically and histologically normal) colonic mucosal in patients with adenomas compared to the control subjects. Importantly, EIBS progressively increased when approaching a neoplastic lesion. Indeed, EIBS was noticeable about 30 cm from the location of the adenoma and progressed at 10 cm from the lesion and at the site of the lesion itself. It is this property of EIBS that may guide an endoscopist to identify high-risk colonic segments. EIBS in superficial tissue was observed even for locations >30 cm away from an adenoma (in colonic segments other than the one where the adenomas were found) (Fig. 6(a)). For comparison, EIBS in subsuperficial tissue was more localized and was observed only for distances <30 cm (Fig. 6(b)). This is consistent with our ex vivo data demonstrating that the spatial extend of EIBS decreases with tissue depth. Finally, Fig. 6(c) demonstrates that hyperplastic polyps do not result in EIBS outside their extend. This is also a promising result as it indicates that the absence of EIBS outside a polyp can be used to determine if a polyp is adenomatous or hyperplastic during the colonoscopy or other endoscopic procedure.

The performance characteristics of EIBS gradient to distinguish colonic segments with and without advanced adenomas are shown in Table 2. These characteristics are encouraging, particularly since because no other currently available technique enables sensing presence of adenomas by analysis of tissue outside the spatial extend of an adenoma. While characterizing the age of an adenoma is not possible, factoring the age of the person may be useful since as one ages microvascular blood content goes down in controls (neoplasia free).

**Table 2: EIBS localization of adenomas in is by in vivo assessment of EIBS 10-30 cm from an na**

| Sensitivity | 100% |
|---|---|
| Specificity | 70% |

The diagnostic performance of EIBS is also superior to conventional CRC screening techniques. For instance, a recent study demonstrated that FOBT and fecal DNA analysis had a sensitivity of 10.8% and 18.2%, respectively, and the sensitivity and positive predictive value of flexible sigmoidoscopy was reported to be only 52% and 6%, respectively. Furthermore, the analysis of our in vivo data showed that there was minor variation in microvascular blood content among the three colonic segments in control subjects, males vs. females, and patients of different age (from 40 to 80 years old). The accuracy of EIBS-based colonoscopy guidance may be improved by accounting for these variations.

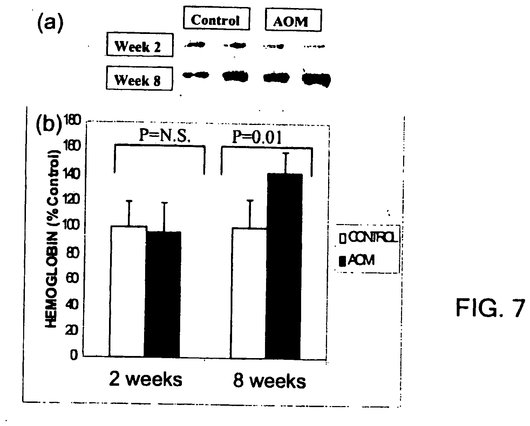

*Non-optics Confirmation of EIBS.* We also wanted to confirm EIBS by use of a non-optics methodology. We used Western blotting and evaluated blood content in the mucosa/submucosa in AOM-treated and control rats. The distal colons of AOM-treated and age-matched control rats were gently scraped. Mucosal homogenates were made and 25 μg of protein was separated on a 10% SDS-PAGE gel, transferred to PDVF membranes and blocked with 5% non-fat milk. Membranes were probed with a polyclonal antibody to hemoglobin (1:300 dilution/overnight at 4C, Santa Cruz Biotechnology) and xerograms were developed with enhanced chemiluminescence and quantitated with a laser densitometer. One clear band at the appropriate molecular weight was noted (68kDa) as shown in Fig. 7(a). This band did not appear on any negative controls (including lysates of two colon cancer cell lines HT-29 and HCT-116 and rat samples probed with secondary antibody alone). Quantitation of the immunoblot analysis (relative to age-matched controls) is demonstrated in Fig. 7(b). As can be seen, there is a significant increase in hemoglobin content in the distal colon at 8 weeks (p=0.01). Blots were stripped and probed for β-actin to confirm uniform protein loading (data not shown).

This provides critical non-optics corroboration of EIBS. Moreover, it underscores the relative lack of sensitivity of non-optics based technologies. For instance, while the 8-week data are significant, the increase is much less dramatic than the 3-fold augmentation of EIBS noted with spectroscopy. Moreover, mucosal blood content analysis failed to reveal a difference at 2 weeks despite highly significant changes seen with spectroscopic analysis. We believe that this dramatic sensitivity of spectroscopic analysis is related in part to its ability to precisely assay just the vasculature. Mucosal scrapings, on the other hand, no matter how gentle, probably samples some of the larger blood vessels in the submucosa. Since we believe that EIBS in the histologically normal mucosa is primarily a microvascular phenomenon, assaying the deeper larger blood vessels can easily obscure the subtle early changes in the microvasculature.

## FURTHER APPLICATIONS OF EIBS

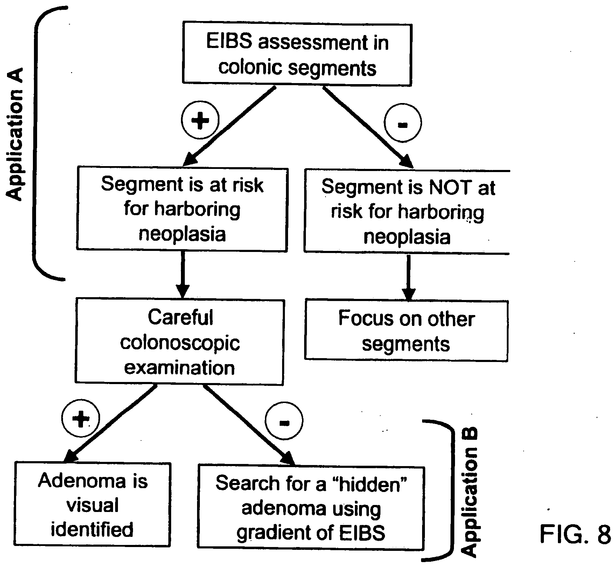

Using 4D-ELF or 2D-ELF (as described hereinafter) and polarization gated spectroscopy, this EIBS biological phenomenon in colonic mucosa in early CRC can be used for early stage detection of lesions. Importantly, spatially, EIBS extended outside the location of a neoplastic lesion (within at least -1/3 of colon from the lesion and beyond, depending on the depth of tissue) and its magnitude increased in the proximity to adenomas. Thus, our data showed that EIBS allowed remarkably accurate determination as to whether a given colonic segment harbors adenoma and could be used to indicate to an endoscopist the proximity of an adenoma. The methodology is to use EIBS to reduce colonoscopic miss rate (15-20% for adenomas and 6-12% for advanced adenomas) by guiding colonoscopy. We propose various applications of EIBS, two of which are illustrated as Application A and Application B in Fig. 8.

*Application A:* EIBS assessed from a given colonic segment will signal endoscopist that this segment is at risk for harboring adenomas and requires more rigorous colonoscopic evaluation. If a segment is not at-risk as determined by EIBS measurements, an endoscopist may make decision to focus on other colonic segments that may require more intense examination.

*Application B:* If adenoma is not readily visualized within this segment, increase magnitude of EIBS with approaching a lesion will guide an endoscopist in search for a hidden neopalsia.

*Other usages of EIBS, Application C*: EIBS can be assessed from distal colon during flexible sigmoidoscopy to assist in detection of the presence of adenomas and tumors in the proximal colon. As is known, a sigmoidoscopy is similar to a colonoscopy but examines only the lower colon and rectum. In such a procedure, the EIBS probe can be used in addition to the sigmoidoscopy probe to obtain the EIBS data. Furthermore, EIBS can be assessed from the rectum either via flexible sigmoidoscopy, a stand-alone fiber-optic probe, or a probe as part of an endoscopic device to assist in detection of the presence of adenomas and tumors in the other parts of colon.

*Other usages of EIBS, Application D:* EIBS can be assessed during colonoscopy, flexible sigmoidoscopy, or other endoscopic procedures to predict the development of future precancerous or cancerous lesions and, thus, assist in determining the schedule (e.g., frequency and time intervals) of future colonoscopies or flexible sigmoidoscopy procedures for a given patient.

Other usages of EIBS, in addition to the usages described above are within the intended scope of the invention, and particularly in conjunction with other diagnostic methods.

In addition to 4D-ELF and polarization gated spectroscopy, other spectroscopic techniques such as, 2D-ELF, enhanced backscattering and low-coherence enhanced backscattering (LEBS) spectroscopy, and OCT can also be used to practice the present invention.

For example, EIBS can be used in conjunction with a screening colonoscopy, in which case the EIBS probe can be used in addition to the colonoscopy probe to obtain the EIBS data.

**PROBE EMBODIMENTS**

In one embodiment, the probe projects a beam of light to a target that has tissues with blood circulation therein. At least one spectrum of light scattered from the target is then measured, and blood supply information related to the target is obtained from the measured at least one spectrum. The obtained blood supply information comprises data related to at least one of blood content, blood oxygenation, blood flow and blood volume.

The probe can be used to obtain different optical measurements. According to one embodiment, it may be used to obtain a first set of the blood supply information from a first location of the target and then obtain a second set of the blood supply information from a second location of the target. The first set of the blood supply information at a first location of the target and the second set of the blood supply information at a second location of the target can then be compared to determine the status of the target. One can compare the data to indicate whether the tumor or lesion exists at all by comparison to previously established microvascular blood content values from patients who harbor neoplasia and from those who are neoplasia free.

In one embodiment, a probe apparatus comprises a light source configured and positioned to project a beam of light to a target; and means for measuring at least one spectrum of light scattered from the target; and means for obtaining blood supply information related to the target from the measured at least one spectrum.

The probe apparatus may further comprise a detector that obtains a first set of the blood supply information at a first location of the target. The same detector can be used to obtain a second set of the blood supply information at a second location of the target. Spectral data which is then obtained from the probe apparatus is analyzed and used to determine whether the tissue that has been inspected is abnormal, as described herein.

In one embodiment, at least one spectrum of light scattered from the target is measured by a fiber optic probe according to the present invention, wherein the fiber optic probe comprises a polarization-gated fiber optic probe configured to detect the blood content information. The light source comprises an incoherent light source (such as a xenon lamp).

In one embodiment, the fiber optic probe includes a proximal end portion, an opposite, distal portion, and a body portion with a longitudinal axis defined between the proximal end portion and the distal portion. The body portion is formed with a cavity along the longitudinal axis. At least one first type of fiber is used for delivering a beam of energy to a target, wherein the at least one first type fiber is at least partially positioned within the cavity of the body portion. An optical element is positioned at the proximal end portion and configured to focus the beam of energy to the target. At least one second type fiber is used for collecting scattered energy from the target, wherein the at least one second type fiber is at least partially positioned within the cavity of the body portion.

The fiber optic probe may further comprise at least one linear polarizer optically coupled to the at least one first type fiber and the at least one second type fiber and positioned proximate to the proximal end portion, and wherein the optical element is positioned at the proximal end portion and configured to focus the scattered energy from the target to the at least one linear polarizes for the at least one second type fiber to collect.

The optical element comprises at least one of a ball lens, a graded refractive index lens, an aspheric lens, cylindrical lens, convex-convex lens, and plano-convex lens, although preferably just a single lens is used. Lenses other than these above-mentioned lenses can also be used. It is further noted that different lenses can be used to assist in discriminating measurements and to achieve different tissue penetration depths. Thus, for example, to achieve the shortest penetration depth, a lens can be positioned at the focal distance from the end of the light-collecting fibers with the fibers positioned symmetrically around the axis of the lens. This configuration further increases the intensity of collected light, particularly when a probe is at a distance from tissue, and provides improved stability of the signals collected by the probe in terms of different distances from tissue (if a probe is not in contact with tissue) and pressures exerted by the probe onto tissue (if a probe is in contact with tissue). Shorter penetration depth can also be achieved by using a lens with a shorter focal distance, smaller numerical aperture of the illumination and/or collection fibers, and larger distance between illumination and collection fiber. In principle, penetration depths from a few tens of microns to a few millimeters can be achieved by choosing a proper combination of these probe characteristics.

The at least one first type fiber comprises an illumination fiber, wherein the illumination fiber is optically coupled to the light source.

The at least one second type fiber can also be formed with one or more collection fibers, wherein the one or more collection fibers are optically coupled to an imaging spectrograph and a CCD at the distal end portion, which imaging spectrograph is used to obtain an image of the target. The body portion comprises a tubing.

The following further details of the preferred embodiments that will further describe the invention.

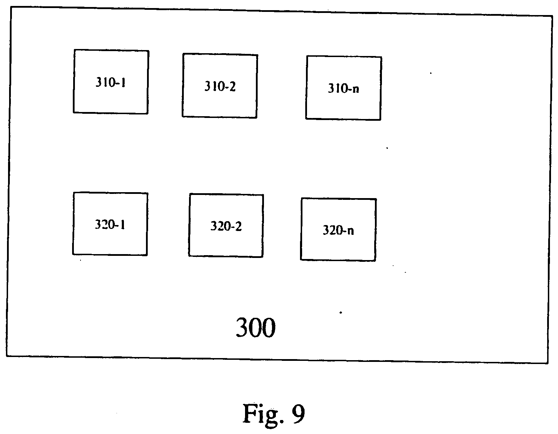

The present invention provides for a probe kit 300, illustrated in Fig. 9 that contains a plurality of interchangeable probe tips 310-1 to 310-n and a plurality of interchangeable optical transmission elements 320-1 to 320-n, where n is an integer greater than 1. Different combinations of these allow for a variety of depth selectivity.

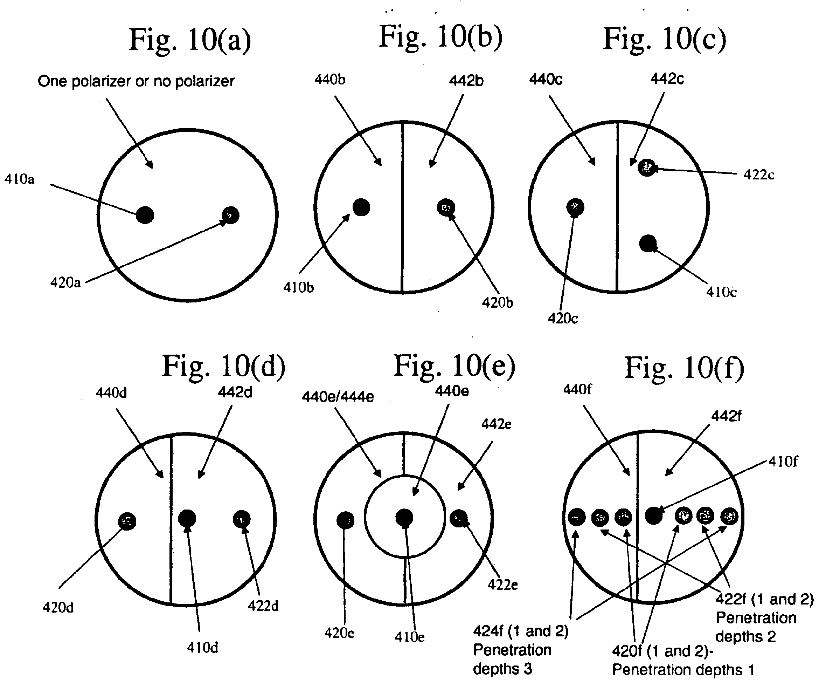

Figs 10(a)-(j) illustrate various configurations of the probe according to the present invention. Figs. 10(a) - (e) illustrate probe configurations that have a single depth selectivity based upon the various characteristics of the components included. Fig 10a shows an embodiment in which there is only a single delivery fiber 410a and a single collection fiber 420a. There may be a polarizer 440a. Fig. 10b is similar to Fig. 10a, but further includes the usage of two polarizers 440b and 442b. Figs. 10c, 10d and 10e illustrate versions with two collection fibers 420c, 422c; 420d, 422d; and 420e, 422e, respectively. In each of these embodiments there are two or three polarizers, 440c and 442c; 440d and 442d ; and 440e, 442e and 444e as shown, respectively, in various configurations relative to the optical fibers.

Figs. 10(f)-(j) illustrate probe configurations in which a single probe, including both the probe tip and the transmission delivery element, can have more than one depth selectivity.

In Fig. 10(f) there exist pairs of collection fibers, and each pair has the same collection depth. Thus collection pair 420f1 and 420f2 have penetration depth 1, collection pair 422f1 and 422f2 have penetration depth 2, and collection pair 424f1 and 424f2 have penetration depth 3. Each of the fibers in each pair is spaced the same distance from the delivery optical fiber 410f. There are two polarizers 440f and 442f as shown.

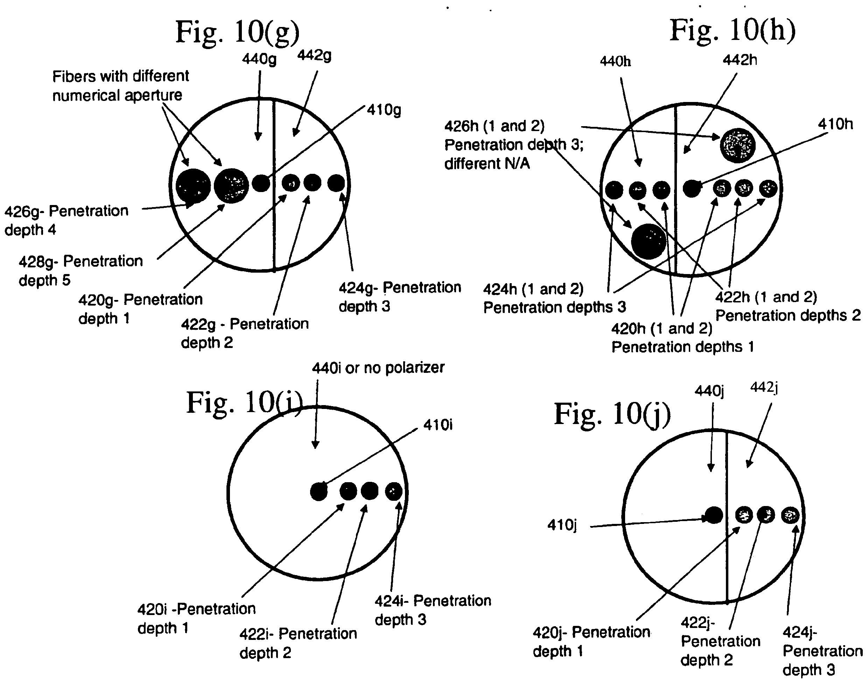

In Fig. 10(g) there is not a collection pair, but rather individual collection fibers 420g, 422g, 424g, 426g and 428g, that each has a different spacing from the delivery optical fiber 410g. Certain of the collection optical fibers have a different numerical aperture than the others, shown as 426g and 428g. There are two polarizers 440g and 442g as shown.

The Fig. 10(h) embodiment is similar to the Fig. 10(f) embodiment, except at the penetration depth 3 there is an additional fiber pair that includes collection optical fibers 426h1 and and 426h4, each of which has a numerical aperture different that the other collection pair at penetration depth 3. There are two polarizers 440h and 442h as shown.

Fig. 10(i) illustrates a probe having a delivery fiber 410i, and three collection fibers 420i, 422i and 424i, each spaced a different distance from the delivery fiber 410i. There is either no polarizer or one polarizer (not labeled).

Fig. 10(j) illustrates a probe that is same as the probe illustrated in Fig. 10(i) except that it includes two polarizers 440(j) and 442(j), rather than none or one polarizer.

The following discussion sets forth the light path for a three-fiber, two polarizer version of a probe, such as illustrated in Fig. 10(b)-(e). A lamp/light source emits unpolarized light. This light is coupled into a delivery fiber 410. Unpolarized light emerges from this fiber 410 and passes through the first polarizer 420 and becomes linearly polarized. This light is diverging with angle of divergence depending on the numerical aperture (NA) of the fiber 410. Typical NA is about 0.22, which means that the angle of divergence is ~25 degrees. Fibers with NA's between 0.1 and 0.5 are also available. This polarized but diverging beam then passes through a lens 430, gets collimated, and impinges upon tissue. The lens 430 is positioned at a focal distance from the fibers 410 and 440. Two collection fibers 440 collect the light that interacts with, such as by backscattering, the tissue. The spot on tissue surface, which is formed such that the light that emerges from tissue from this spot can reach and can get collected by one of the collection fibers 440, will be referred to as the "collection spot" for a given collection fiber 440. If tissue surface is in the focal plane of the lens 430 (GRIN lenses typically have the focal planes coinciding with their surfaces) all illumination and collection spots coincide. One of the collection fibers 440 shares the same polarizer 420 with the delivery fiber 410 and the other fiber 440 is "behind" a second polarizer 450 with the axis of polarization orthogonal (or, to be more general, just different) to the axis of polarization of the first polarizer 420. The light that interacts, such as by being backscattered, from tissue has both polarization components. Each of these polarizers 420 and 450, selects light polarized in a particular way and only this light reaches the corresponding collection fiber 440. The first fiber 440 collects light that is polarized along the same direction as the incident light. This is co-polarized light. The other fiber 440 collects the cross-polarized light. On the other (proximal) ends, the light transmitted through the collection fibers 440 is coupled into a spectrometer and a detector (not shown). The spectrometer and a detector can be a single linear array detector (one for each fiber) or an imaging spectrometer and a CCD (which is more expensive). The detector records the spectrum of light intensity from each fiber, which becomes the co-polarized ($I_{\parallel}$) and cross-polarized signals/spectra ($I_{\perp}$). These spectra are then transmitted to a computer or a CPU. The computer can process these spectral data. Four different spectral curves can be looked at: 1) Differential polarization (or what is called polarization-gated) signal is calculated as $I_{\parallel}-I_{\perp}$; 2) The total (or arbitrarily polarized) signal is found as $I_{\parallel}+I_{\perp}$; 3) Co-polarized signal $I_{\parallel}$; and 4) Cross-polarized signal $I_{\perp}$. Each of these four signals is preferentially sensitive to tissue up to its own penetration depth. In principle, one does not have to use two polarizers and measure both co- and cross-polarized signals. If shallow penetration depth is not desired, one can use just a single polarizer and only one collection fiber (co-polarized signal only), two polarizers and collect cross-polarized signal, no polarizer and collect $I_{\parallel}+I_{\perp}$, etc.

Specific characteristic combinations for two different probes 100 are provided below.

Example 1: Ball lens probe

Ball lens diameter: 2 mm,
focal length: 1.1 mm,
fiber core diameter: 200 microns,
numerical aperture (NA): 0.22,
distance between illumination and detection fibers: 0.5 mm,
spot size on tissue surface: 0.5 mm,
output (incident on tissue) beam divergence: 5 deg,
outer diameter of the probe: 2.6 mm.

Example 2: GRIN lens probe

GRIN lens diameter: 1.8 mm,
focal length: 2.4 mm,
fiber core diameter: 200 um,
NA: 0.22,
distance between illumination and detection fibers: 0.7 mm,
spot size: 0.7 mm,
output beam divergence: 3 deg,
outer diameter of the probe: 2.5 mm.

In addition to modifying characteristics of the probe tips and the optical transmission elements as mentioned above, there are other characteristics that can be modified.

These modifications include altering where the end of the collection fiber is relative to the delivery fiber in order to get different angular ranges for this depth selective probe. Positioning fibers away from the focal distance is essentially equivalent to using fibers with a larger diameter that are positioned in the focal plane, which in turn is equivalent to a greater divergence of incident or collected light beams. Greater divergence results in addition of both longer and shorter light paths inside tissue. Overall, in most cases this will result in deeper penetration. It should be noted, however, that by "defocusing" the probe, the "defocusing" configuration is less efficient in terms of intensity the probe collects.

Another characteristic is the distance to the tissue surface. This distance can be controlled by choosing a proper spacer between the lens and the tissue surface. (As discussed above, most GRIN lenses have their focal plane coinciding with their sides but this is not necessarily the case with other lens types. If other lenses are used, one can put a spacer to distance the probe from tissue.) If this distance is different from the focal length, penetration depth is greater.

FURTHER ASPECTS OF THE INVENTION

In one aspect, the EIBS in vivo blood content measurement apparatus contains three major parts: the illumination arm, the detection arm, and the probe. A particular combination of these three major parts can be chosen based on functional, cost and other considerations. Possible designs of the probe are discussed above. Below, a number of possible combinations of devices used for illumination and detection are discussed. For illustrative purposes, these designs are based on a probe design with two collection channels for a co- and a cross-polarized signals. These apparatus designs can be easily modified for other number of collection fibers.

1. Illumination options:

a) (a) White light arclamp

i. Advantages: smooth broadband spectrum
ii. Disadvantages: poor coupling efficiency, higher cost, shorter lifetime, poor stability unless a power stabilizer is employed

b) White Light Emitting Diode (WLED)

i. Advantages: high efficiency, low cost, long lifetime, good stability

ii. Disadvantages: irregular emission spectra, poor coupling, lower intensity

c) Broadband-spectrum laser or a number of lasers with output at different wavelengths

i. Advantages: excellent coupling, high intensity

ii. Disadvantages: limited spectral sampling, speckle, high cost

d) Color LEDs - a number of LEDs of specific spectral ranges

i. Advantages: sequential puling, no speckle, low cost

ii. Disadvantages: limited spectral sampling, lower intensity [000124]

2. Detection and system design

a) Three spectrometers with reference signal - Use three single channel fiber spectrometers: one for each polarization, and one reference which samples the source. The signal from a target is normalized in respect to the reference measurement so that the instability of the sources spectrum and intensity would not effect measurement.

i. Advantages: best stability

ii. Disadvantages: highest cost and system complexity

b) Two spectrometers, no reference - two single channel fiber spectrometers are used to detect each polarization state. The source spectrum and intensity is assumed to stable.

i. Advantages: lowest cost, simplest

ii. Disadvantages: increased likelihood of error due to source variations unless a stable light source is used or measurements from tissue are self-normalized (e.g. the diagnostic observable is the ratio of the blood content values from the two depths)

c) Two spectrometers, intensity reference - Two single channel fiber spectrometers are used to detect each polarization state. The source intensity is monitored with a simple point detector.

i. Advantages: low cost, does not require a stable current light source

ii. Disadvantages: could be sensitive to spectral changes in the source

d) Two intensity detectors with or without a reference detector - can be used with illumination provided by a set of color LEDs or laser sources for sequential acquisition of the spectral data.

i. Advantages: low cost

ii. Disadvantages: limited spectral resolution

Preferred embodiments of the above combinations include 1) A system with WLED, two spectrometers and an intensity reference can provide an optimal compromise between the cost and functionality. 2) A system with WLED, two spectrometers and no reference is an optimal configuration for applications where sufficient diagnostic information can be obtained by computing a ratio of observables (e.g. blood content) from different collection channels or depth. 3) A system with a set of at least three color LEDs and a single intensity detector for sequential illumination and acquisition is an example of a low-cost configuration for applications when cost is a critical consideration.

## Claims

1. An apparatus configured for emitting broadband light obtained from a light source onto a tissue and receives interacted light that is obtained from interaction of the broadband light with the tissue, comprising:

a probe, the probe including:

an end adapted for insertion into the human body;

a delivery optical fiber having a delivery numerical aperture for transmitting the broadband light obtained from a light source, the light delivery optical fiber having a light delivery end adapted for emission of the broadband light and a source connection end adapted for connection to the light source;

at least one collection optical fiber having a collection numerical aperture, the collection optical fiber having a light collection end that receives the interacted light and a receiver connection end, wherein the light collection end is substantially aligned with and at a predetermined distance from the light delivery end of the delivery optical fiber;

a lens that is spaced substantially about one focal length of the lens from the light collection end of the collection optical fiber and from the light delivery end of the delivery optical fiber and,

a receiver coupled to the at least one collection optical fiber at the receiver connection end, and adapted for estimating a characteristic indicative of at least blood content or blood flow in the at least one of mucosal and submucosal tissue based on the received interacted light; and

an indicator electrically coupled to said receiver and adapted for providing an indication based on said estimated characteristic;

**characterized in that** said indicator is adapted for providing the indication of the presence of increased blood supply in the illuminated target tissue based on a comparison with a prior measurement of another region of the at least one of mucosal and submucosal tissue.

2. The apparatus according to claim 1, wherein the probe is contained within one of an endoscope and a sigmoidoscope.

3. Apparatus according to claim 1 wherein the probe includes two and only two collection optical fibers.

4. Apparatus according to claim 3 wherein the two collection optical fibers have the same collection numerical aperture and the light collection end of each collection optical fiber is substantially aligned with and substantially at a same predetermined distance from the light delivery end of the delivery optical fiber.

5. Apparatus according to claim 4 further including first and second polarizers disposed between the lens and the delivery and collection optical fibers, the first polarizer providing polarization of the emitted broadband light and the interacted light that is directed to one of the two collection optical fibers and the second polarizer providing polarization of the interacted light that is directed to the other of the two collection optical fibers, and wherein the inclusion of the first and second polarizers and the polarization of each are further ones of the selected plurality of characteristics.

6. The apparatus of claim 2, further comprising a display device coupled to a receiver, said indicator adapted to provide an indication related to at least one of blood content and blood flow at the illuminated tissue location, and wherein the display device displays by symbols, color, alphanumerics, light, sound and similar techniques and combinations thereof at least one of the presence of increased blood supply and a comparison of consecutive or non-consecutive measurements of a characteristic of increased blood supply and indicate the direction a probe should be moved to predict the increase or decrease of the measurement of a characteristic of blood supply.

7. An apparatus according to claim 2,
wherein the delivery optical fiber and the collection optical fiber and the lens are adapted to collect the interacted light at a collection spot on a surface of the tissue that is within the focal plane of the lens, and wherein the interacted light collected results from interactions with the at least one of the mucosal and submucosal tissue that are substantially at a predetermined penetration depth below the collection spot, and wherein the predetermined penetration depth is obtained in part due to a selected plurality of characteristics, the selected plurality of characteristics including selection of the focal length of the lens and at least one further characteristic from the delivery optical fiber, the collection optical fiber, or the lens.

8. The apparatus according to claim 7, wherein the at least one further characteristic is a characteristic of one of the delivery optical fiber and the collection optical fiber, and is either (1) a type of the delivery optical fiber, (2) a type of the collection optical fiber, (3) the delivery and collection numerical apertures, (4) the substantial alignment of the light delivery end of the delivery optical fiber and the light collection end of the collection optical fiber, (5) the predetermined distance between the light delivery end of the delivery optical fiber and the light collection end of the collection optical fiber and (6) a type of lens.

9. The apparatus according to claim 7, wherein the predetermined penetration depth corresponds to a depth of one of the mucosal and submucosal tissue.

10. The apparatus according to claim 7, wherein there are two and only two collection optical fibers.

11. The apparatus according to claim 10, wherein the two collection optical fibers have the same collection numerical aperture and the light collection end of each collection optical fiber is substantially aligned with and substantially at a same predetermined distance from the light delivery end of the delivery optical fiber.

12. The apparatus according to claim 11, further including first and second polarizers disposed between the lens and the delivery and collection optical fibers, the first polarizer providing polarization of the emitted broadband light and the interacted light that is directed to one of the two collection optical fibers and the second polarizer providing polarization of the interacted light that is directed to the other of the two collection optical fibers, and wherein the inclusion of the first and second polarizers and the polarization of each are further ones of the selected plurality of characteristics.

13. The apparatus according to claim 12, wherein a depth of the blood content in at least one of musosal and submucosal tissue that is detected is between 0 and 250 microns.

14. The apparatus according to claim 2, further including a receiver that includes a linear array CCD detector.

15. The apparatus according to claim 12, wherein the light source emits at least two wavelength ranges of light.

16. The apparatus according to claim 15, wherein the two wavelength ranges are such that one of them includes 542, 555 and 576 nm wavelengths and the other includes wavelengths longer than 576 nm.

17. The apparatus according to claim 7, wherein the probe further includes a polarizer disposed between the lens and the delivery and collection optical fibers, the polarizer allowing for at least one of adjustment of polarization of the emitted broadband light and for polarization of the interacted light.

18. An apparatus according to claim 7, wherein the delivery optical fiber and the collection optical fiber are formed as one interchangeable optical transmission element having a device connection end and a detection end, the detection end including the light delivery end and the light connection end of the delivery optical fiber and the collection optical fiber, respectively;

   wherein the lens is formed as one interchangeable probe tip assembly, and
   wherein there is at least one of (a) a plurality of interchangeable probe tip assemblies including the one interchangeable probe tip assembly and (b) a plurality of interchangeable optical transmission elements including the one interchangeable optical transmission element,
   wherein each of the plurality of interchangeable probe tip assemblies and each of the plurality of interchangeable optical transmission elements having a different characteristic selected to assist with the detection of the interacted light at different tissue penetration depths so that coupling of a particular interchangeable fiber transmission element to a particular interchangeable probe tip assembly will provide for detection of interacted light at a particular tissue penetration depth.

19. The apparatus according to claim 18, wherein there is a plurality of probe tip assemblies.

20. The apparatus according to claim 19, wherein at least some of the interchangeable probe tip assemblies further include a polarizer disposed between the lens and the delivery and collection optical fibers, the polarizer providing for a further different characteristic of the at least some probe tip assemblies.

21. The apparatus according to claim 20, wherein the polarizer is adapted for at least one of adjustment of polarization of the emitted broadband light and for polarization of the interacted light.

22. The apparatus according to claim 19, where a further different characteristic is a distance between the device connection end and a focal plane of the lens for at least some of the plurality of probe tip assemblies.

23. The apparatus according to claim 19, where a further different characteristic is a distance between a focal plane of the lens and a surface of the tissue surface.

24. The apparatus according to claim 22, wherein an outer spacer is used to ensure the distance between the focal plane of the lens and the surface of the tissue surface for at least some of the plurality of probe tip assemblies.

25. The apparatus according to claim 19, wherein one lens in one of the probe tip assemblies has a larger focal length than another lens in another of the probe tip assemblies to obtain a larger spot size of the emitted broadband light.

26. The apparatus according to claim 2,

wherein the delivery optical fiber and the collection optical fiber are formed as one interchangeable optical transmission element having a device connection end and a detection end, the detection end including the light delivery end and the light connection end of the delivery optical fiber and the collection optical fiber, respectively; wherein the lens is formed as one interchangeable probe tip assembly, and
wherein there is at least one of (a) a plurality of interchangeable probe tip assemblies including the one interchangeable probe tip assembly, and (b) a plurality of interchangeable optical transmission elements including the one interchangeable optical transmission element.

**Patentansprüche**

1. Vorrichtung, die konfiguriert ist zum Emittieren von Breitbandlicht, das aus einer Lichtquelle erhalten wird, auf ein Gewebe und die in Wechselwirkung tretendes Licht empfängt, das aus einer Wechselwirkung des Breitbandlichts mit dem Gewebe erhalten wird, umfassend:

eine Sonde, die Sonde beinhaltend:

ein Ende, das zur Einführung in den menschlichen Körper ausgebildet ist;
eine optische Abgabefaser, die eine numerische Abgabeapertur zum Übertragen des Breitbandlichts, das aus einer Lichtquelle erhalten wird, aufweist, wobei die optische Faser zur Lichtabgabe ein Lichtabgabeende, das ausgebildet ist zur Emission des Breitbandlichts, und ein Quellverbindungsende aufweist, das zur Verbindung mit der Lichtquelle ausgebildet ist;
mindestens eine optische Aufnahmefaser, die eine numerische Aufnahmeapertur aufweist, wobei die optische Aufnahmefaser ein Lichtaufnahmeende, das das in Wechselwirkung tretende Licht empfängt, und ein Verbindungsende des Empfängers aufweist, wobei das Lichtaufnahmeende im Wesentlichen an dem Lichtabgabeende der optischen Abgabefaser ausgerichtet ist und sich in einem vorgegebenen Abstand davon befindet;
eine Linse beinhaltet, die im Wesentlichen um eine Brennweite der Linse von dem Lichtaufnahmeende der optischen Aufnahmefaser und von dem Lichtabgabeende der optischen Abgabefaser beabstandet ist, und
einen Empfänger, der mit der mindestens einen optischen Aufnahmefaser an dem Verbindungsende des Empfängers gekoppelt ist und ausgebildet ist zum Schätzen eines Merkmals, das mindestens auf Blutgehalt in dem mindestens einen aus Mucosal- und Submucosalgewebe hinweist, basierend auf dem empfangenen, in Wechselwirkung tretenden Lichts; und

einen Indikator, der elektrisch mit dem Empfänger gekoppelt ist und zum Bereitstellen eines Hinweises basierend auf dem geschätzten Merkmal ausgebildet ist;
**dadurch gekennzeichnet, dass** der Indikator ausgebildet ist zum Bereitstellen des Hinweises auf das Vorhandensein einer verstärkten Blutzufuhr in dem beleuchteten Zielgewebe, basierend auf einem Vergleich mit einer vorherigen Messung eines anderen Bereichs des mindestens einen aus Mucosal- und Submucosalgewebe.

2. Vorrichtung nach Anspruch 1, wobei die Sonde innerhalb eines aus einem Endoskop und einem Sigmoidoskop enthalten ist.

3. Vorrichtung nach Anspruch 1, wobei die Sonde zwei und nur zwei optische Aufnahmefasern beinhaltet.

4. Vorrichtung nach Anspruch 3, wobei die zwei optischen Aufnahmefasern die gleiche numerische Aufnahmeapertur aufweisen und das Lichtaufnahmeende jeder optischen Aufnahmefaser im Wesentlichen an dem Lichtabgabeende der optischen Abgabefaser ausgerichtet ist und sich im Wesentlichen in einem gleichen vorgegebenen Abstand davon befindet.

5. Vorrichtung nach Anspruch 4, ferner beinhaltend erste und zweite Polarisatoren, die zwischen der Linse und den optischen Abgabe- und Aufnahmefasern angeordnet sind, wobei der erste Polarisator eine Polarisierung des emittierten Breitbandlichts und des in Wechselwirkung tretenden Lichts bereitstellt, das zu einem der beiden optischen Aufnahmefasern geleitet wird, und der zweite Polarisator eine Polarisierung des in Wechselwirkung tretenden Lichts bereitstellt, das zu dem anderen der beiden optischen Aufnahmefasern geleitet wird, und wobei die Aufnahme des ersten und des zweiten Polarisators und die jeweilige Polarisierung weitere aus der ausgewählten Vielzahl von Merkmalen sind.

6. Vorrichtung nach Anspruch 2, ferner umfassend eine Anzeigevorrichtung, die mit einem Empfänger gekoppelt ist, wobei der Indikator ausgebildet ist, um eine Angabe betreffend mindestens eines aus Blutgehalt und Blutfluss an der beleuchteten Gewebestelle bereitzustellen, und wobei die Anzeigevorrichtung durch Symbole, Farbe, alphanumerische Zeichen, Licht, Geräusch und ähnliche Techniken und Kombinationen davon mindestens eines aus dem Vorhandensein einer verstärktem Blutzufuhr und eines Vergleichs aufeinanderfolgender oder nicht aufeinanderfolgender Messungen eines Merkmals verstärkter Blutzufuhr anzeigt und die Richtung angibt, in die eine Sonde bewegt werden sollte, um den Anstieg oder die Abnahme der Messung eines Merkmals von Blutzufuhr vorherzusagen.

7. Vorrichtung nach Anspruch 2, wobei die optische Abgabefaser und die optische Aufnahmefaser und die Linse ausgebildet sind, um das in Wechselwirkung tretende Licht an einer Sammelstelle auf einer Oberfläche des Gewebes zu sammeln, die sich innerhalb der Brennebene der Linse befindet, und wobei das gesammelte, in Wechselwirkung tretende Licht aus Wechselwirkungen mit dem mindestens einen aus dem Mucosal- und Submucosalgewebe resultiert, die sich im Wesentlichen in einer vorgegebenen Penetrationstiefe unterhalb der Aufnahmestelle befinden, und wobei die vorgegebene Penetrationstiefe teilweise aufgrund einer ausgewählten Vielzahl von Merkmalen erhalten wird, die ausgewählte Vielzahl von Merkmalen eine Auswahl der Brennweite der Linse und mindestens ein weiteres Merkmal aus der optischen Abgabefaser, der optischen Aufnahmefaser oder der Linse beinhaltet.

8. Vorrichtung nach Anspruch 7, wobei das mindestens eine weitere Merkmal ein Merkmal von einer aus der optischen Abgabefaser und der optischen Aufnahmefaser ist und entweder (1) eine Art der optischen Abgabefaser, (2) eine Art der optischen Aufnahmefaser, (3) die numerischen Abgabe- und Aufnahmeöffnungen, (4) die wesentliche Ausrichtung des Lichtabgabeendes der optischen Abgabefaser und des Lichtaufnahmeendes der optischen Aufnahmefaser, (5) der vorgegebene Abstand zwischen dem Lichtabgabeende der optischen Abgabefaser und dem Lichtaufnahmeende der optischen Aufnahmefaser oder (6) eine Art von Linse ist.

9. Vorrichtung nach Anspruch 7, wobei die vorbestimmte Penetrationstiefe einer Tiefe von einem des Mucosal- und des Submucosalgewebes entspricht.

10. Vorrichtung nach Anspruch 7, wobei es zwei und nur zwei optische Aufnahmefasern gibt.

11. Vorrichtung nach Anspruch 10, wobei die zwei optischen Aufnahmefasern die gleiche numerische Aufnahmeapertur aufweisen und das Lichtaufnahmeende jeder optischen Aufnahmefaser im Wesentlichen an dem Lichtabgabeende der optischen Abgabefaser ausgerichtet ist und sich im Wesentlichen in einem gleichen vorgegebenen Abstand davon befindet.

12. Vorrichtung nach Anspruch 11, ferner beinhaltend erste und zweite Polarisatoren, die zwischen der Linse und den optischen Abgabe- und Aufnahmefasern angeordnet sind, wobei der erste Polarisator eine Polarisierung des emittierten Breitbandlichts und des in Wechselwirkung tretenden Lichts bereitstellt, das zu einem der beiden optischen Aufnahmefasern geleitet wird, und der zweite Polarisator eine Polarisierung des in Wechselwirkung tretenden Lichts bereitstellt, das zu dem anderen der beiden optischen Aufnahmefasern geleitet wird, und wobei die Aufnahme des ersten und des zweiten Polarisators und die Polarisierung jedes davon weitere aus der ausgewählten Vielzahl von Merkmalen sind.

13. Vorrichtung nach Anspruch 12, wobei eine Tiefe des Blutgehalts in mindestens einem aus dem Mucosal- und des Submucosalgewebes, das erkannt wird, zwischen 0 und 250 Mikron beträgt.

14. Vorrichtung nach Anspruch 2, ferner beinhaltend einen Empfänger, der einen linearen CCD Arraydetektor beinhaltet.

**15.** Vorrichtung nach Anspruch 12, wobei die Lichtquelle mindestens zwei Wellenlängenbereiche des Lichts emittiert.

**16.** Vorrichtung nach Anspruch 15, wobei die zwei Wellenlängenbereiche derart sind, dass einer von ihnen Wellenlängen von 542, 555 und 576 nm beinhaltet und der andere Wellenlängen beinhaltet, die länger als 576 nm sind.

**17.** Vorrichtung nach Anspruch 7, wobei die Sonde ferner einen Polarisator beinhaltet, der zwischen der Linse und den optischen Abgabe- und Aufnahmefasern angeordnet ist, wobei der Polarisator mindestens eines aus einer Einstellung der Polarisation des emittierten Breitbandlichts und einer Polarisation des in Wechselwirkung tretenden Lichts ermöglicht.

**18.** Vorrichtung nach Anspruch 7, wobei die optische Abgabefaser und die optische Aufnahmefaser als ein austauschbares optisches Übertragungselement ausgebildet sind, das ein Vorrichtungsverbindungsende und ein Erkennungsende aufweist, wobei das Erkennungsende das Lichtabgabeende und das Lichtverbindungsende der optischen Abgabefaser beziehungsweise der optischen Aufnahmefaser beinhaltet;
wobei die Linse als eine austauschbare Sondenspitzenanordnung ausgebildet ist und
wobei mindestens eines vorhanden ist aus (a) einer Vielzahl von austauschbaren Sondenspitzenanordnungen, die die eine austauschbare Sondenspitzenanordnung beinhalten, und (b) einer Vielzahl von austauschbaren optischen Übertragungselementen, die das eine austauschbare optische Übertragungselement beinhalten,
wobei jede aus der Vielzahl von austauschbaren Sonnenspitzenanordnungen und jede aus der Vielzahl von austauschbaren optischen Übertragungselementen ein unterschiedliches Merkmal aufweisen, das ausgewählt ist, um die Erkennung des in Wechselwirkung tretenden Lichts bei verschiedenen Gewebepenetrationstiefen zu unterstützen, sodass eine Kopplung eines bestimmten austauschbaren Faserübertragungselements mit einer bestimmten austauschbaren Sondenspitzenanordnung eine Erkennung von in Wechselwirkung tretendem Licht bei einer bestimmten Gewebepenetrationstiefe bereitstellt.

**19.** Vorrichtung nach Anspruch 18, wobei eine Vielzahl von Sondenspitzenanordnungen vorhanden ist.

**20.** Vorrichtung nach Anspruch 19, wobei mindestens einige der austauschbaren Sondenspitzenanordnungen ferner einen Polarisator beinhalten, der zwischen der Linse und den optischen Abgabe- und Aufnahmefasern angeordnet ist, wobei der Polarisator ein weiteres unterschiedliches Merkmal der mindestens einigen Sondenspitzenanordnungen bereitstellt.

**21.** Vorrichtung nach Anspruch 20, wobei der Polarisator für mindestens eines aus einer Einstellung der Polarisation des emittierten Breitbandlichts und einer Polarisation des in Wechselwirkung tretenden Lichts ausgebildet ist.

**22.** Vorrichtung nach Anspruch 19, wobei ein weiteres unterschiedliches Merkmal ein Abstand zwischen dem Vorrichtungsverbindungsende und einer Brennebene für mindestens einige aus der Vielzahl von Sondenspitzenanordnungen ist.

**23.** Vorrichtung nach Anspruch 19, wobei ein weiteres unterschiedliches Merkmal ein Abstand zwischen einer Brennebene der Linse und einer Oberfläche der Gewebeoberfläche ist.

**24.** Vorrichtung nach Anspruch 22, wobei ein äußerer Abstandshalter verwendet wird, um den Abstand zwischen der Brennebene der Linse und der Oberfläche der Gewebeoberfläche für mindestens einige aus der Vielzahl von Sonnenspitzenanordnungen zu gewährleisten.

**25.** Vorrichtung nach Anspruch 19, wobei eine Linse in einer der Sondenspitzenanordnungen eine größere Brennweite aufweist als eine andere Linse in einer anderen der Sondenspitzenanordnungen, um eine größere Fleckgröße des emittierten Breitbandlichts zu erhalten.

**26.** Vorrichtung nach Anspruch 2,
wobei die optische Abgabefaser und die optische Aufnahmefaser als ein austauschbares optisches Übertragungselement ausgebildet sind, das ein Vorrichtungsverbindungsende und ein Erkennungsende aufweist, wobei das Erkennungsende das Lichtabgabeende und das Lichtverbindungsende der optischen Abgabefaser beziehungsweise der optischen Aufnahmefaser beinhaltet;
wobei die Linse als eine austauschbare Sondenspitzenanordnung ausgebildet ist und
wobei mindestens eines vorhanden ist aus (a) einer Vielzahl von austauschbaren Sondenspitzenanordnungen, die die eine austauschbare Sondenspitzenanordnung beinhalten, und (b) einer Vielzahl von austauschbaren optischen Übertragungselementen, die das eine austauschbare optische Übertragungselement beinhalten.

**Revendications**

1. Appareil configuré pour émettre une lumière à large bande obtenue à partir d'une source lumineuse sur un tissu et recevoir une lumière ayant interagi qui est obtenue par l'interaction de la lumière à large bande avec le tissu, comprenant :

   une sonde, la sonde comprenant :

   une extrémité conçue pour une insertion dans le corps humain ;
   une fibre optique de distribution présentant une ouverture numérique de distribution pour émettre la lumière à large bande obtenue à partir d'une source lumineuse, la fibre optique de distribution de lumière présentant une extrémité de distribution de lumière conçue pour une émission de la lumière à large bande et une extrémité de connexion à une source conçue pour une connexion à la source lumineuse ;
   au moins une fibre optique de collecte présentant une ouverture numérique de collecte, la fibre optique de collecte présentant une extrémité de collecte de lumière qui reçoit la lumière ayant interagi et une extrémité de connexion à un récepteur, l'extrémité de collecte de lumière étant sensiblement alignée sur l'extrémité de distribution de lumière de la fibre optique de distribution et à une distance prédéterminée de celle-ci ;
   une lentille qui est espacée sensiblement d'environ une longueur focale de la lentille vis-à-vis de l'extrémité de collecte de lumière de la fibre optique de collecte et vis-à-vis de l'extrémité de distribution de lumière de la fibre optique de distribution, et

   un récepteur couplé à l'au moins une fibre optique de collecte au niveau de l'extrémité de connexion à un récepteur, et conçu pour estimer une caractéristique indicative d'au moins la teneur sanguine ou l'écoulement sanguin dans l'au moins un de tissus muqueux et sous-muqueux sur la base de la lumière ayant interagi reçue ; et
   un indicateur couplé électriquement audit récepteur et conçu pour fournir une indication sur la base de ladite caractéristique estimée ;
   **caractérisé par le fait que**
   ledit indicateur est conçu pour fournir l'indication de la présence d'apport en sang accru dans le tissu cible éclairé sur la base d'une comparaison avec une mesure antérieure d'une autre région de l'au moins un de tissus muqueux et sous-muqueux.

2. Appareil selon la revendication 1, dans lequel la sonde est contenue dans l'un d'un endoscope et d'un sigmoïdoscope.

3. Appareil selon la revendication 1, dans lequel la sonde comprend deux et uniquement deux fibres optiques de collecte.

4. Appareil selon la revendication 3, dans lequel les deux fibres optiques de collecte présentent la même ouverture numérique de collecte et l'extrémité de collecte de lumière de chaque fibre optique de collecte est sensiblement alignée sur l'extrémité de distribution de lumière de la fibre optique de distribution et sensiblement à une même distance prédéterminée de celle-ci.

5. Appareil selon la revendication 4, comprenant en outre des premier et second polariseurs disposés entre la lentille et les fibres optiques de distribution et de collecte, le premier polariseur fournissant une polarisation de la lumière à large bande émise et de la lumière ayant interagi qui est dirigée vers l'une des deux fibres optiques de collecte et le second polariseur fournissant une polarisation de la lumière ayant interagi qui est dirigée vers l'autre des deux fibres optiques de collecte, et dans lequel l'inclusion des premier et second polariseurs et la polarisation de chacun sont d'autres de la pluralité sélectionnée de caractéristiques.

6. Appareil selon la revendication 2, comprenant en outre un dispositif d'affichage couplé à un récepteur, ledit indicateur étant conçu pour fournir une indication associée à au moins l'un de la teneur sanguine et de l'écoulement sanguin au niveau de l'emplacement de tissu éclairé, et dans lequel le dispositif d'affichage affiche, par des symboles, une couleur, des caractères alphanumériques, une lumière, un son et des techniques similaires et des combinaisons de celles-ci, au moins l'une de la présence d'apport en sang accru et d'une comparaison de mesures consécutives ou non consécutives d'une caractéristique d'apport en sang accru et indiquer la direction selon laquelle une sonde devrait être déplacée pour prédire l'augmentation ou la diminution de la mesure d'une caractéristique d'apport en sang.

**7.** Appareil selon la revendication 2, dans lequel la fibre optique de distribution et la fibre optique de collecte et la lentille sont conçues pour collecter la lumière ayant interagi au niveau d'un point de collecte sur une surface du tissu qui est dans le plan focal de la lentille, et dans lequel les résultats collectés de lumière ayant interagi provenant d'interactions avec l'au moins un de tissus muqueux et sous-muqueux qui sont sensiblement à une profondeur de pénétration prédéterminée au-dessous du point de collecte, et dans lequel la profondeur de pénétration prédéterminée est obtenue en partie en raison d'une pluralité sélectionnée de caractéristiques, la pluralité sélectionnée de caractéristiques comprenant la sélection de la longueur focale de la lentille et au moins une autre caractéristique à partir de la fibre optique de distribution, de la fibre optique de collecte ou de la lentille.

**8.** Appareil selon la revendication 7, dans lequel l'au moins une autre caractéristique est une caractéristique de l'une de la fibre optique de distribution et de la fibre optique de collecte, et est l'un parmi (1) un type de la fibre optique de distribution, (2) un type de la fibre optique de collecte, (3) les ouvertures numériques de distribution et de collecte, (4) l'alignement substantiel de l'extrémité de distribution de lumière de la fibre optique de distribution et de l'extrémité de collecte de lumière de la fibre optique de collecte, (5) la distance prédéterminée entre l'extrémité de distribution de lumière de la fibre optique de distribution et l'extrémité de collecte de lumière de la fibre optique de collecte et (6) un type de lentille.

**9.** Appareil selon la revendication 7, dans lequel la profondeur de pénétration prédéterminée correspond à une profondeur de l'un des tissus muqueux et sous-muqueux.

**10.** Appareil selon la revendication 7, dans lequel il y a deux et uniquement deux fibres optiques de collecte.

**11.** Appareil selon la revendication 10, dans lequel les deux fibres optiques de collecte présentent la même ouverture numérique de collecte et l'extrémité de collecte de lumière de chaque fibre optique de collecte est sensiblement alignée sur l'extrémité de distribution de lumière de la fibre optique de distribution et sensiblement à une même distance prédéterminée de celle-ci.

**12.** Appareil selon la revendication 11, comprenant en outre des premier et second polariseurs disposés entre la lentille et les fibres optiques de distribution et de collecte, le premier polariseur fournissant une polarisation de la lumière à large bande émise et de la lumière ayant interagi qui est dirigée vers l'une des deux fibres optiques de collecte et le second polariseur fournissant une polarisation de la lumière ayant interagi qui est dirigée vers l'autre des deux fibres optiques de collecte, et dans lequel l'inclusion des premier et second polariseurs et la polarisation de chacun sont d'autres de la pluralité sélectionnée de caractéristiques.

**13.** Appareil selon la revendication 12, dans lequel une profondeur de la teneur sanguine dans au moins l'un de tissus muqueux et sous-muqueux qui est détectée est entre 0 et 250 micromètres.

**14.** Appareil selon la revendication 2, comprenant en outre un récepteur qui comprend un détecteur CCD à matrice linéaire.

**15.** Appareil selon la revendication 12, dans lequel la source lumineuse émet au moins deux plages de longueurs d'onde de lumière.

**16.** Appareil selon la revendication 15, dans lequel les deux plages de longueurs d'onde sont telles que l'une d'elles comprend des longueurs d'onde de 542, 555 et 576 nm et l'autre comprend des longueurs d'onde plus longues que 576 nm.

**17.** Appareil selon la revendication 7, dans lequel la sonde comprend en outre un polariseur disposé entre la lentille et les fibres optiques de distribution et de collecte, le polariseur permettant au moins l'un d'un ajustement de polarisation de la lumière à large bande émise et de polarisation de la lumière ayant interagi.

**18.** Appareil selon la revendication 7, dans lequel la fibre optique de distribution et la fibre optique de collecte sont formées en tant qu'élément d'émission optique interchangeable présentant une extrémité de connexion à un dispositif et une extrémité de détection, l'extrémité de détection comprenant l'extrémité de distribution de lumière et l'extrémité de connexion de lumière de la fibre optique de distribution et la fibre optique de collecte, respectivement ;

dans lequel la lentille est formée en tant que ensemble pointe de sonde interchangeable, et

dans lequel il y a au moins l'un (a) d'une pluralité d'ensembles pointes de sonde interchangeables comprenant ledit ensemble pointe de sonde interchangeable et (b) d'une pluralité d'éléments d'émission optique interchangeables comprenant ledit élément d'émission optique interchangeable,

dans lequel chacun de la pluralité d'ensembles pointes de sonde interchangeables et chacun de la pluralité d'éléments d'émission optique interchangeables présentent une caractéristique différente sélectionnée pour aider à la détection de la lumière ayant interagi à différentes profondeurs de pénétration de tissu de telle sorte que le couplage d'un élément d'émission par fibre interchangeable particulier à un ensemble pointe de sonde interchangeable particulier fournira une détection de lumière ayant interagi à une profondeur de pénétration de tissu particulière.

19. Appareil selon la revendication 18, dans lequel il y a une pluralité d'ensembles pointes de sonde.

20. Appareil selon la revendication 19, dans lequel au moins certains des ensembles pointes de sonde interchangeables comprennent en outre un polariseur disposé entre la lentille et les fibres optiques de distribution et de collecte, le polariseur fournissant une autre caractéristique différente des au moins certains ensembles pointes de sonde.

21. Appareil selon la revendication 20, dans lequel le polariseur est conçu pour au moins l'un d'un ajustement de polarisation de la lumière à large bande émise et de polarisation de la lumière ayant interagi.

22. Appareil selon la revendication 19, dans lequel une autre caractéristique différente est une distance entre l'extrémité de connexion à un dispositif et un plan focal de la lentille pour au moins certains de la pluralité d'ensembles pointes de sonde.

23. Appareil selon la revendication 19, dans lequel une autre caractéristique différente est une distance entre un plan focal de la lentille et une surface de la surface de tissu.

24. Appareil selon la revendication 22, dans lequel un espaceur externe est utilisé pour garantir la distance entre le plan focal de la lentille et la surface de la surface de tissu pour au moins certains de la pluralité d'ensembles pointes de sonde.

25. Appareil selon la revendication 19, dans lequel une lentille dans l'un des ensembles pointes de sonde présente une plus grande longueur focale qu'une autre lentille dans un autre des ensembles pointes de sonde pour obtenir une dimension de point plus grande de la lumière à large bande émise.

26. Appareil selon la revendication 2, dans lequel la fibre optique de distribution et la fibre optique de collecte sont formées en tant qu'élément d'émission optique interchangeable présentant une extrémité de connexion à un dispositif et une extrémité de détection, l'extrémité de détection comprenant l'extrémité de distribution de lumière et l'extrémité de connexion de lumière de la fibre optique de distribution et de la fibre optique de collecte, respectivement ;

dans lequel la lentille est formée en tant qu'ensemble pointe de sonde interchangeable, et

dans lequel il y a au moins l'un (a) d'une pluralité d'ensembles pointes de sonde interchangeables comprenant ledit ensemble pointe de sonde interchangeable, et (b) d'une pluralité d'éléments d'émission optique interchangeables comprenant ledit élément d'émission optique interchangeable.

FIG. 1

110   120

Illumination-
collection angle

142

130

Focal length

Tissue

Illumination/collection spot size

100

Fig. 2(a)

120

140   142

122

110

Fig. 2 (a) shows cross section    Fig. 2(b)

Figure 3

(a) Superficial tissue (obtained from ΔI)

(b) Subsuperficial tissue (obtained from $I_\perp$)

(c) Hb assessment via "absorption band area" in $I_\perp$

(d) Hb assessment via "absorption band intensity" in $I_\perp$

FIG. 5

FIG. 6A

FIG. 6B

FIG. 6C

FIG. 6D

FIG. 7

FIG. 8

```
┌─────────────────────────────────────────────┐
│                                             │
│   ┌────────┐   ┌────────┐   ┌────────┐       │
│   │        │   │        │   │        │       │
│   │ 310-1  │   │ 310-2  │   │ 310-n  │   .   │
│   │        │   │        │   │        │       │
│   └────────┘   └────────┘   └────────┘       │
│                                         .    │
│                                             │
│   ┌────────┐   ┌────────┐   ┌────────┐       │
│   │        │   │        │   │        │       │
│   │ 320-1  │   │ 320-2  │   │ 320-n  │       │
│   │        │   │        │   │        │       │
│   └────────┘   └────────┘   └────────┘       │
│                                    .    .    │
│                                             │
│                   300            .          │
│                                             │
└─────────────────────────────────────────────┘
```

Fig. 9

# Fig. 10(a)

One polarizer or no polarizer

410a

420a

# Fig. 10(b)

440b

442b

410b

420b

# Fig. 10(c)

440c

442c

422c

410c

420c

# Fig. 10(d)

440d

442d

420d

410d

422d

# Fig. 10(e)

440e/444e

440e

442e

422e

420e

410e

424f (1 and 2)
Penetration
depths 3

# Fig. 10(f)

440f

442f

410f

422f (1 and 2)
Penetration
depths 2

420f (1 and 2)-
Penetration depths 1

Fig. 10(g)

Fibers with different numerical aperture

440g  442g

410g

426g- Penetration depth 4

428g- Penetration depth 5

420g- Penetration depth 1

422g - Penetration depth 2

424g- Penetration depth 3

Fig. 10(h)

440h  442h

426h (1 and 2) Penetration depth 3; different N/A

410h

424h (1 and 2) Penetration depths 3

420h (1 and 2) Penetration depths 1

422h (1 and 2) Penetration depths 2

Fig. 10(i)

440i or no polarizer

410i

420i -Penetration depth 1

422i- Penetration depth 2

424i- Penetration depth 3

Fig. 10(j)

440j  442j

410j

420j- Penetration depth 1

422j- Penetration depth 2

424j- Penetration depth 3

EP 2 020 910 B1

1100

1120

Detector
1130

Probe
1110

Computer
1140

Indicator
Device
1150

FIG. 11

1210

1220

1212

1218

1222

1214

1216

Screen
1224

FIG. 12A

Text Screen
1260

"Possible
Suspicious" or
"Negative"
displayed

FIG 12B